# EUROPEAN PATENT SPECIFICATION

(11) **EP 2 348 951 B1**
(45) Date of publication and mention of the grant of the patent: **06.08.2014**
(21) Application number: 09821682.3
(22) Date of filing: 01.10.2009
(51) Int. Cl.: A61B 1/04

(54) **ASSEMBLIES FOR USE WITH ENDOSCOPES AND APPLICATIONS THEREOF**
ANORDNUNGEN ZUR ANWENDUNG MIT ENDOSKOPEN UND IHRE ANWENDUNGEN
ENSEMBLES DESTINÉS À ÊTRE UTILISÉS AVEC DES ENDOSCOPES, ET LEURS APPLICATIONS

(30) Priority: 20.10.2008 US 136978 P; 10.12.2008 US 193605 P; 23.03.2009 WO PCT/IL2009/000322
(43) Date of publication of application: 03.08.2011
(60) Divisional application: 14165202.4
(73) Proprietor: Smart Medical Systems Ltd., 43663 Ra'anana (IL)
(72) Inventor: TERLIUC, Gad, 43465 Ra'anana (IL); LURIA, Gilad, 53419 Givataim (IL); NISSAN, Ori, 52335 Ramat Gan (IL)
(74) Representative: Dericioglu Kurt, Ekin
(86) International application number: PCT/IL2009/000940
(87) International publication number: WO 2010/046891

(56) References cited:
- EP-A1- 1 541 080
- WO-A2-2007/017854
- US-A- 4 517 702
- US-A- 4 898 492
- US-A- 5 135 001
- US-A- 5 259 366
- US-A- 5 762 604
- US-A- 5 938 586
- US-A1- 2004 210 116

## Description

### FIELD OF THE INVENTION

The present invention relates to endoscope systems generally.

### BACKGROUND OF THE INVENTION

The following patent publications and commercially available products are believed to represent the current state of the art:
U.S. Patent Nos. 4,040,413; 4,195,633; 4,453,545; 5,259,366; 6,309,346; 6,461,294; 6,585,639;
U.S. Patent Application publication Nos. 2004/0102681; 2005/0124856; 2005/0125005; 2005/0133453; 2005/0165233; 2006/0111610; 2006/0161044 and 2003/0244361;
Double Balloon Endoscope product, including EN-450T5 enteroscope, TS-13140 overtube and BS-2 front balloon, which interface with balloon pump control BP-20 and 2200 video system, all commercially available from Fujinon Inc., of 10 High Point Drive, Wayne, New Jersey, USA; and,
Single Balloon Endoscope product, including SIF-Q180 enteroscope, ST-SB1 overtube, which interface with balloon pump control OBCU and EVIS EXERA II system video system, all commercially available from Olympus Inc., of 3500 Corporate Parkway Center Valley, PA 18034-0610, USA.
US 5,259,366 discloses a side - mountable sleeve.
US 5,762,604 discloses a balloon with longitudinal edges bond together to form a cylindrical outer wall.

### SUMMARY OF THE INVENTION

The present invention seeks to provide improved assemblies for operation with elongate articles such as endoscopes.

There is thus provided in accordance with a preferred embodiment of the present invention a side-mountable overtube as defined in the appended claims, for use with an endoscope. The overtube including a sleeve formed with a slit, extending entirely along its length, and with a balloon inflation/deflation lumen communicating with a balloon opening at a balloon opening location intermediate along its length, and a selectably inflatable/deflatable balloon mounted onto the sleeve in a manner such that it does not extend entirely across the slit and thereby is side mountable along with the sleeve onto an endoscope, an interior of the balloon communicating with the balloon opening at the balloon opening location for selectable inflation/deflation of the balloon.

Preferably, the inflatable/deflatable balloon, when inflated, defines mutually touching inflated balloon surfaces extending generally along the slit edges. Preferably, the overtube is configured for removable attachment to the endoscope when the balloon is inflated, thereby preventing sliding motion between the overtube and the endoscope. Alternatively, the overtube is configured to permit relative sliding motion between the overtube and the endoscope when the balloon is inflated.

Preferably, the overtube inflatable/deflatable balloon includes at least first and second axial sealing surfaces which extend generally parallel to the slit. Additionally or alternatively, the side-mountable overtube also includes a side lumen extending at least partially along the length of the sleeve and traversing the balloon.

In accordance with another preferred embodiment the side-mountable overtube also includes an instrument channel tube communicating with a rearward portion of the side lumen and an instrument channel port which communicates with an end portion of the instrument channel tube. Additionally or alternatively, the side-mountable overtube also includes a securing mechanism for selectably securing the side-mountable overtube on the endoscope. Preferably, the securing mechanism includes a double-sided adhesive tape adapted to selectably join opposing axial edges of the slit. Preferably, the side-mountable overtube also includes an inflation/deflation functionality operative for selectable inflation and deflation of the balloon.

### BRIEF DESCRIPTION OF THE DRAWINGS

The present invention will be understood and appreciated from the following detailed description, taken in conjunction with the drawings in which:
Figs. 1A and 1B are simplified pictorial illustrations of an endoscope overtube, suitable for use in endoscopic procedures carried out in tubular body portions of patients, constructed and operative in accordance with a preferred embodiment of the present invention in respective open and closed orientations;
Fig. 2 is a simplified exploded pictorial illustration of the endoscope overtube of Figs. 1A & 1B;
Figs. 3A, 3B and 3C are respective simplified pictorial, end view and side view illustrations of a wrap-around balloon employed in the endoscope overtube of Figs. 1A-2;
Figs. 4A, 4B, 4C, 4D and 4E are simplified illustrations of association of the endoscope overtube of Figs. 1A - 2 with a conventional endoscope and a conventional endoscope tool;
Figs. 5A and 5B are simplified pictorial illustrations of an ultrasound overtube assembly constructed and operative in accordance with a preferred embodiment of the present invention in respective unmounted and endoscope-mounted orientations;
Fig. 6 is a simplified exploded pictorial illustration of the ultrasound overtube assembly of Figs. 5A & 5B;
Figs. 7A, 7B and 7C are respective simplified pictorial, end view and side view illustrations of a wrap-around balloon employed in the ultrasound overtube assembly of Figs. 5A - 6;
Fig. 8 is a simplified pictorial, partially cutaway, illustration of the overtube of Figs. 5A - 7C in an operational orientation, associated with an endoscope and located within a tubular body portion of a patient;
Figs. 9A and 9B are simplified pictorial illustrations of an endoscope irrigation and suction overtube constructed and operative in accordance with a preferred embodiment of the present invention in respective open and closed orientations.
Figs. 10A, 10B, 10C, 10D & 10E are simplified pictorial illustrations of use of the overtube of the embodiment of Figs. 1A - 4E in an endoscopic treatment;
Fig. 11A is a simplified, pictorial illustration of a hollow-tube stiffener constructed and operative in accordance with a preferred embodiment of the present invention;
Fig. 11B is a simplified partially sectional pictorial illustration of the stiffener of Fig. 11A fully inserted in an instrument channel of a conventional endoscope;
Fig. 12A is a simplified pictorial illustration of a stiffener having non-uniform stiffness along its length, constructed and operative in accordance with a preferred embodiment of the present invention;
Fig. 12B is a simplified partially sectional pictorial illustration of the stiffener of Fig. 12A fully inserted in an instrument channel of a conventional endoscope;
Fig. 13A is a simplified pictorial illustration of a balloon catheter having non-uniform stiffness along its length, constructed and operative in accordance with a preferred embodiment of the present invention;
Fig. 13B is a simplified partially sectional pictorial illustration of the balloon catheter of Fig. 13A fully inserted in an instrument channel of a conventional endoscope;
Fig. 14A is a simplified partially sectional illustration of an instrument channel extender constructed and operative in accordance with a preferred embodiment of the present invention;
Fig. 14B is a simplified partially sectional, partially pictorial illustration of the use of the instrument channel extender of Fig. 14A with a conventional endoscope in accordance with a preferred embodiment of the present invention;
Fig. 15A is a simplified partially sectional illustration of a catheter assembly incorporating an instrument channel extender, constructed and operative in accordance with a preferred embodiment of the present invention; and
Fig. 15B is a simplified partially sectional, partially pictorial illustration of the use of the catheter assembly of Fig. 15A with a conventional endoscope in accordance with a preferred embodiment of the present invention.

### DETAILED DESCRITION OF PREFERRED EMBODIMENTS

The terms "endoscope" and "endoscopy" are used throughout in a manner somewhat broader than their customary meaning and refer to apparatus and methods which operate within body cavities, passageways and the like, such as, for example, the small intestine, the large intestine, arteries and veins. Although these terms normally refer to visual inspection, as used herein they are not limited to applications which employ visual inspection and refer as well to apparatus, systems and methods which need not necessarily involve visual inspection.

The term "forward" refers to the remote end of an endoscope, accessory or tool furthest from the operator or to a direction facing such remote end.

The term "rearward" refers to the end portion of an endoscope, accessory or tool closest to the operator, typically outside an organ or body portion of interest or to a direction facing such end portion.

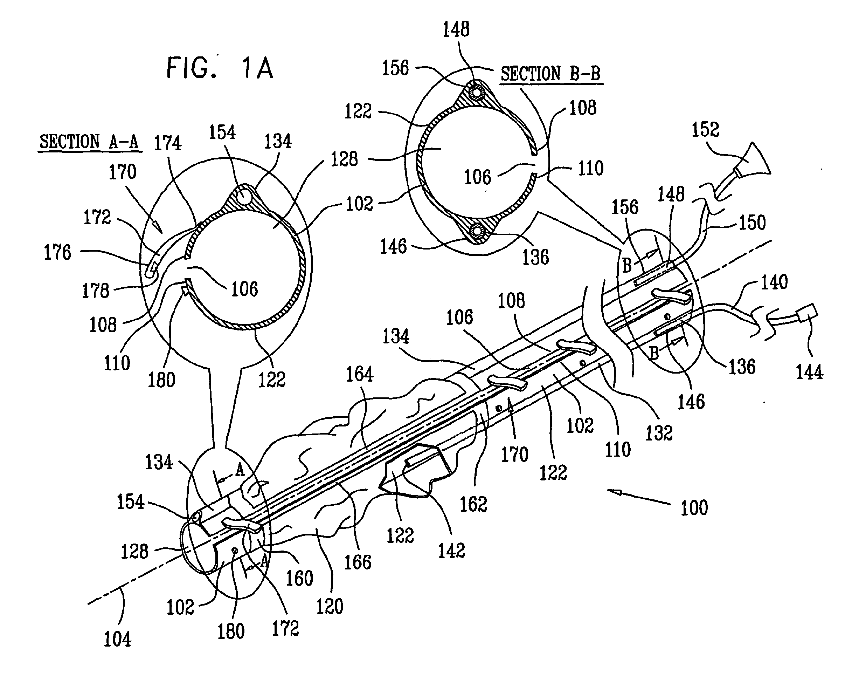

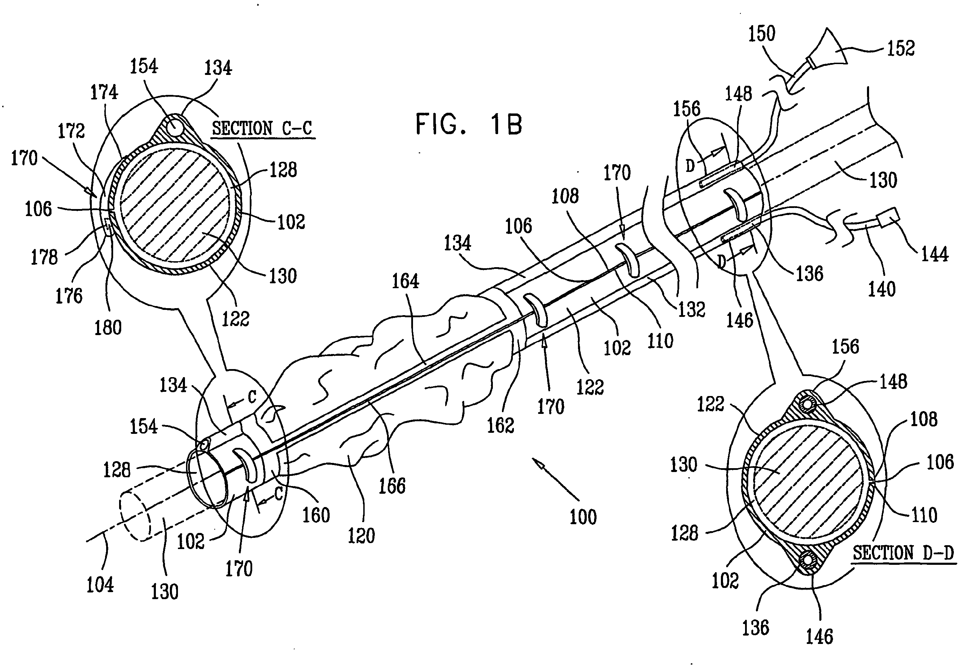

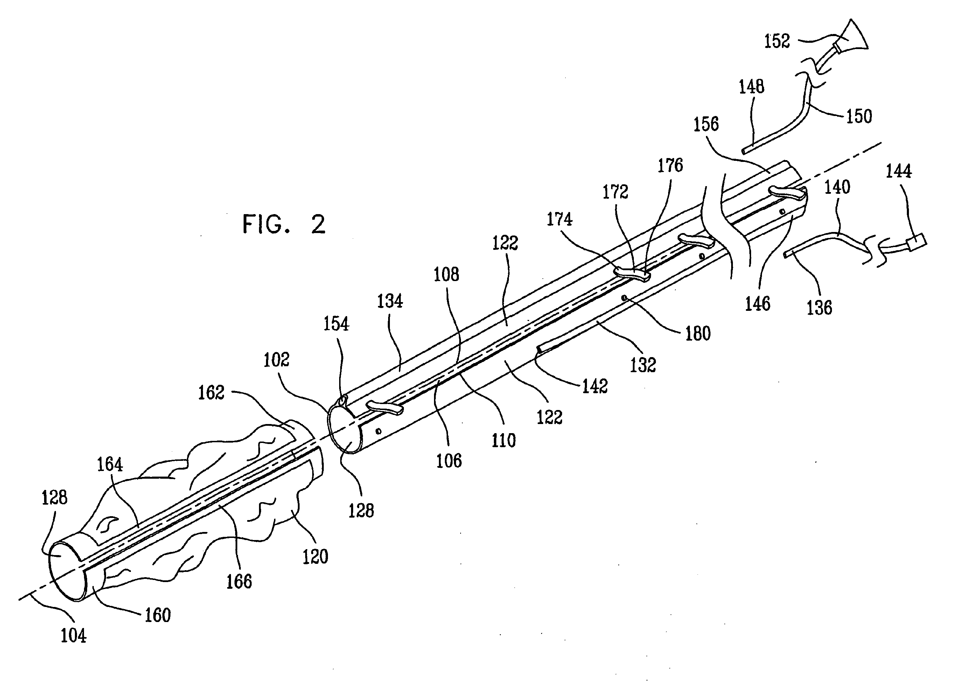

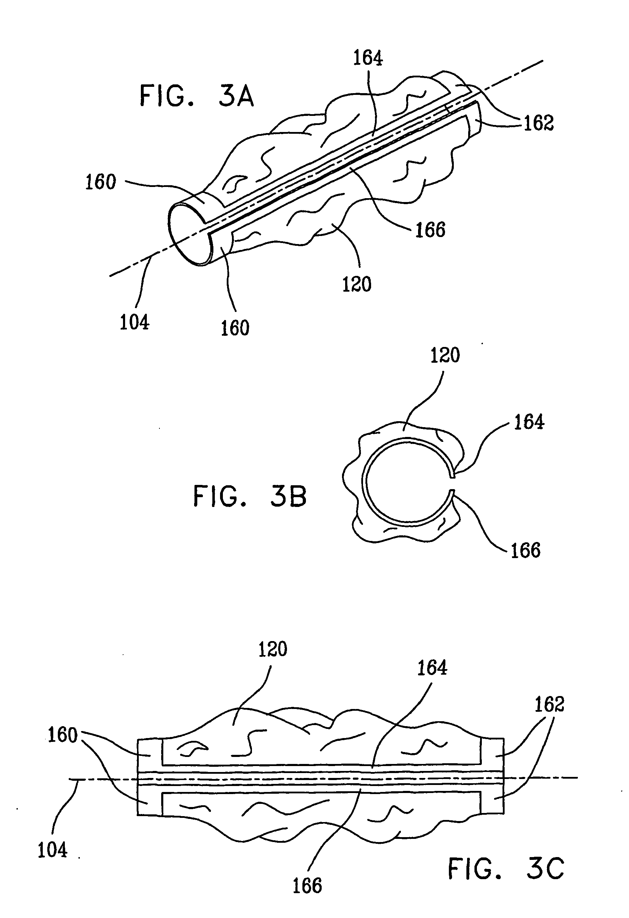

Reference is now made to Figs. 1A & 1B, which are simplified pictorial illustrations of an endoscope overtube constructed and operative in accordance with a preferred embodiment of the present invention in respective open and closed orientations; Fig. 2, which is a simplified exploded pictorial illustration of the endoscope overtube of Figs. 1A & 1B and Figs. 3A, 3B & 3C, which are respective simplified pictorial, end view and side view illustrations of a wrap-around balloon employed in the endoscope overtube of Figs. 1A - 2.

As seen in Figs. 1A - 2, there is provided an endoscope overtube 100 constructed and operative in accordance with a preferred embodiment of the present invention including a generally non axially compressible, tubular generally cylindrical sleeve 102 arranged about a longitudinal axis 104. Sleeve 102 is preferably slit axially, as indicated by reference numeral 106, thus defining axial slit edges 108 and 110 and thus is side openable and closable. A selectably inflatable/deflatable wrap-around balloon 120, preferably a near fully circumferential wrapped balloon, is mounted over part of an outer surface 122 of sleeve 102.

Sleeve 102 preferably has a main lumen 128 for accommodating an endoscope 130, and first and second side lumens 132 and 134 which are circumferentially spaced from each other along outer surface 122 of sleeve 102. Endoscope 130 is preferably a conventional endoscope, such as a VSB-3430K video enteroscope, a EG-2930K video gastroscope or a EC-3430LK video colonoscope, which are connectable to an endoscopy console such as a console including a EPK-1000 video processor and a SONY LMD-2140MD medical grade flat panel LCD monitor, all commercially available from Pentax Europe GmbH, 104 Julius-Vosseler St., 22523 Hamburg, Germany.

First side lumen 132 accommodates a forward portion 136 of a flexible balloon inflation/deflation tube 140 and extends partially along the length of sleeve 102, outwardly of main lumen 128, to an opening 142 underlying and in fluid communication with the interior of balloon 120. Preferably, flexible tube 140 extends from a connector 144 outside of sleeve 102 and forward portion 136 thereof extends partially along and inside a rearward portion 146 of first side lumen 132 and is fixedly and sealingly attached thereto as by a suitable adhesive, so as to provide a sealed inflation/deflation pathway therewith.

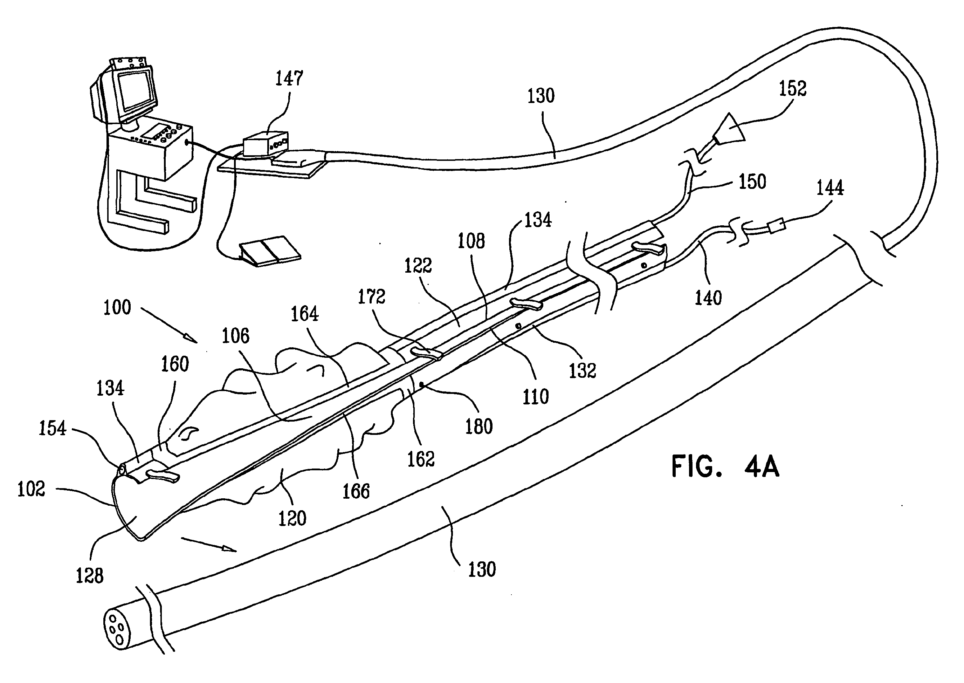

Preferably, connector 144 is connectable to a balloon inflation/deflation system 147 (Figs. 4A - 4E). System 147 may be similar to the inflation control assembly described in details in applicant/assignee's PCT patent application PCT/IL2007/000600. It is appreciated that system 147 may be operative for supply of gas or liquid to balloon 120 as needed, and for drainage of the gas or liquid therefrom. Alternatively to system 147, a syringe or other inflation/deflation mechanism may be employed.

Second side lumen 134 accommodates a forward portion 148 of a flexible instrument channel tube 150 which extends from a tool insertion port 152 outside of sleeve 102. Preferably, second side lumen 134 extends along the entire length of sleeve 102, along outer surface 122 thereof and underlying balloon 120 from a rear edge of sleeve 102 to an open end 154. Instrument channel tube 150 extends from port 152, partially along and inside a rearward portion 156 of second side lumen 134 and is fixedly attached thereto as by a suitable adhesive.

Balloon 120 is preferably a preformed, flexible element, having a generally cylindrical configuration when assembled onto sleeve 102 and securely mounted onto endoscope 130 (Fig. 1B). Balloon 120 includes peripheral sealing surfaces which are preferably adhesively joined or heat welded onto outer surface 122 of sleeve 102. The peripheral sealing surfaces preferably include respective forward and rearward circumferential collar sealing surfaces 160 and 162 and first and second axial sealing surfaces 164 and 166 which extend parallel to slit edges 108 and 110. Preferably, balloon 120 is mounted onto sleeve 102 in a manner such that it does not extend entirely across slit 106 of sleeve 102 and thereby is side mountable along with sleeve 102 onto an endoscope, such as endoscope 130, as will be described hereinbelow with reference to Figs. 4A - 4D.

It is appreciated that the generally axial slit 106 of sleeve 102 may be straight or curved, such as a straight slit parallel to longitudinal axis 104, a spiral slit along longitudinal axis 104, or a sinusoidal slit. The forward edge of sleeve 102 is preferably smooth and rounded so as to avoid damage to tissue under examination during in vivo inspection of a generally tubular body portion such as the intestine.

Preferably, sleeve 102 is shorter than endoscope 130 and is of a typical length of approximately 90 - 160 cm and has an inner diameter of approximately 10 - 14 mm and an outer diameter of approximately 12 -16 mm, so as to be readily slidable over a conventional endoscope. Preferably, sleeve 102 is configured for mounting over endoscopes of various diameters, such as in the range of 9.5 - 13 mm. Yet preferably, the thickness of sleeve 102 is in the range of 0.3 - 2 mm, and may be constant or varying along its length.

In accordance with a preferred embodiment of the present invention, the forward portion of sleeve 102 underlying balloon 120 is relatively rigid, thereby not allowing radially inward deformation of sleeve 102 during inflation of balloon 120, so as not to inhibit slidable motion of endoscope 130 through sleeve 102 when balloon 120 is inflated. It is appreciated that such a configuration of overtube 100 which permits relative sliding motion between overtube 100 and endoscope 130 when balloon 120 is inflated and preferably anchoring overtube 100 to the internal walls of the colon, may be employed by an operator for advancing and retracting endoscope 130 in the colon by respectively pushing and pulling it through overtube 100.
Alternatively, the forward portion of sleeve 102 underlying balloon 120 is highly flexible, thereby allowing radially inward deformation of sleeve 102 during inflation of balloon 120, so as to grip endoscope 130 and bind sleeve 102 to endoscope 130 when balloon 120 is inflated, thereby preventing sliding motion therebetween.

It is appreciated that sleeve 102 is relatively flexible, thereby being able to conform to bending of endoscope 130 onto which it is slidably mounted, and is yet sufficiently rigid so as to allow its sliding over endoscope 130 by pushing it forward at a rearward end thereof. Sleeve 102 may be formed of any suitable material such as silicone, PEBAX®, PVC or polyurethane. In accordance with a preferred embodiment of the present invention, the inner surface of sleeve 102 is formed of a low-friction material, such as thin and flexible internal TEFLON® tube or a hydrophilic coating, so as to allow low resistance sliding of sleeve 102 over an endoscope in a bent orientation.

It is appreciated that in accordance with a preferred embodiment of the present invention balloon 120 is generally inflatable, and can be inflated to a diameter about 3 - 10 times larger than its diameter when not inflated. In accordance with a preferred embodiment of the present invention, useful for small intestine endoscopy, the diameter of balloon 120 when fully inflated is in the range of 35 - 45 mm. Preferably, inflation of balloon 120 to a diameter less than 45 mm may be achieved using relatively low pressure, such as in the range of 10-20 millibars (1000 - 2000 Pa).

In another specific embodiment, useful for large intestine endoscopy, the diameter of balloon 120, when fully inflated, is in the range of 4 - 6 centimeters. In a further embodiment, also useful for large intestine endoscopy, the diameter of balloon 120, when fully inflated, is six centimeters. Preferably, inflation of balloon 120 to a diameter less than six centimeters may be achieved using relatively low pressure, such as in the range of 20 - 40 millibars (2000 - 4000 Pa).

It is appreciated that in accordance with a preferred embodiment of the present invention, useful for in vivo inspection of a generally tubular body portion having a variable cross-sectional diameter, the expansion diameter range of balloon 120, when mounted onto sleeve 102, is larger than the maximum cross-sectional diameter of the generally tubular body portion, thereby enabling engagement of expanded balloon 120 with the interior surface of the generally tubular body portion, and anchoring of sleeve 102 thereto. Preferably, balloon 120 is a relatively soft, highly compliant balloon, operative to at least partially conform to the shape of the interior surface of the generally tubular body portion when in engagement therewith.

It is appreciated that balloon 120 may be formed of suitable well-known stretchable materials such as latex, flexible silicone, or highly flexible nylon. Balloon 120 may be compliant, semi-compliant or generally non-compliant. In the latter two cases, it may be formed of a material such as polyurethane which is less flexible, stretchable and compliant than latex, flexible silicone and highly flexible nylon. Preferably, the diameter of balloon 120 is sufficient to ensure tight anchoring at any part of the generally tubular body portion.

A plurality of latches 170 are provided for selectably securely mounting overtube 100 onto endoscope 130 and are distributed along the length of overtube 100 other than underlying balloon 120. These latches preferably each include an arm portion 172, which is joined at one end 174 thereof, to outer surface 122 of sleeve 102 adjacent one of edges 108 and 110 of slit 106 as by adhesive or heat welding. At an opposite end 176 of each arm there is provided a first attachment portion 178 which removably engages a corresponding second attachment portion 180 mounted adjacent an opposite one of edges 108 and 110. In the illustrated embodiment, the arm portions 172 are attached adjacent edges 108 and the first attachment portion 178 is a recess which mates with a corresponding protrusion defining the second attachment portion 180. Any other suitable arrangement may be employed.

It is appreciated that overtube 100 may be selectably securely mounted onto endoscope 130 by employing any other suitable securing mechanism, such as double-sided adhesive tape or a zipper-like mechanism for joining edges 108 and 110.

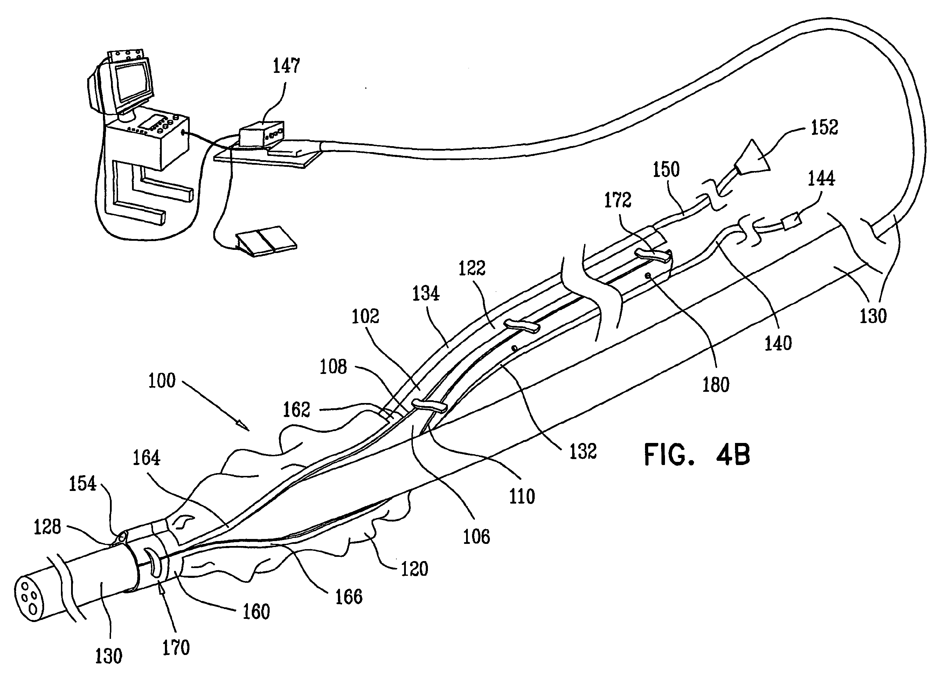

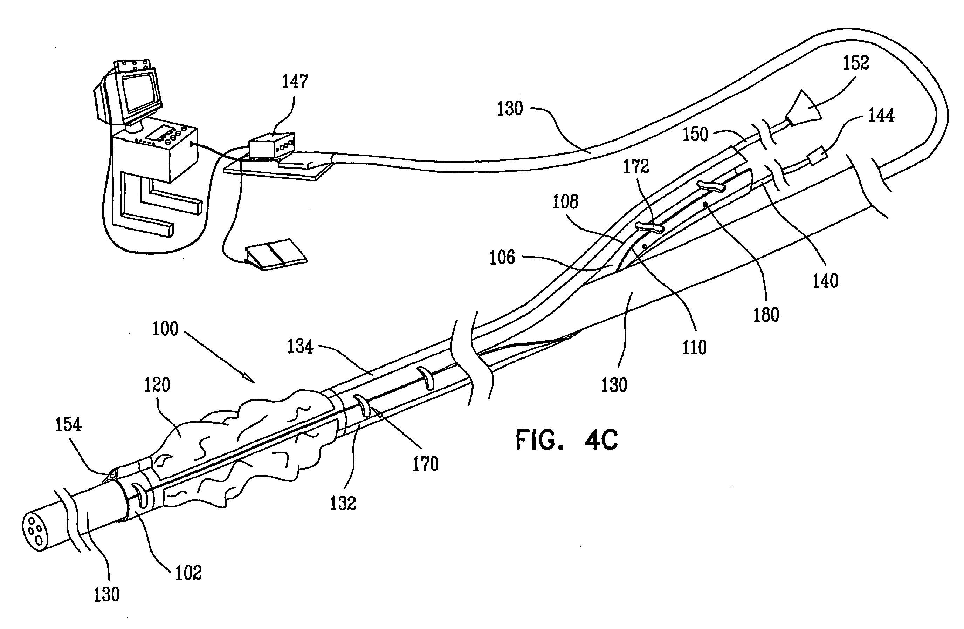

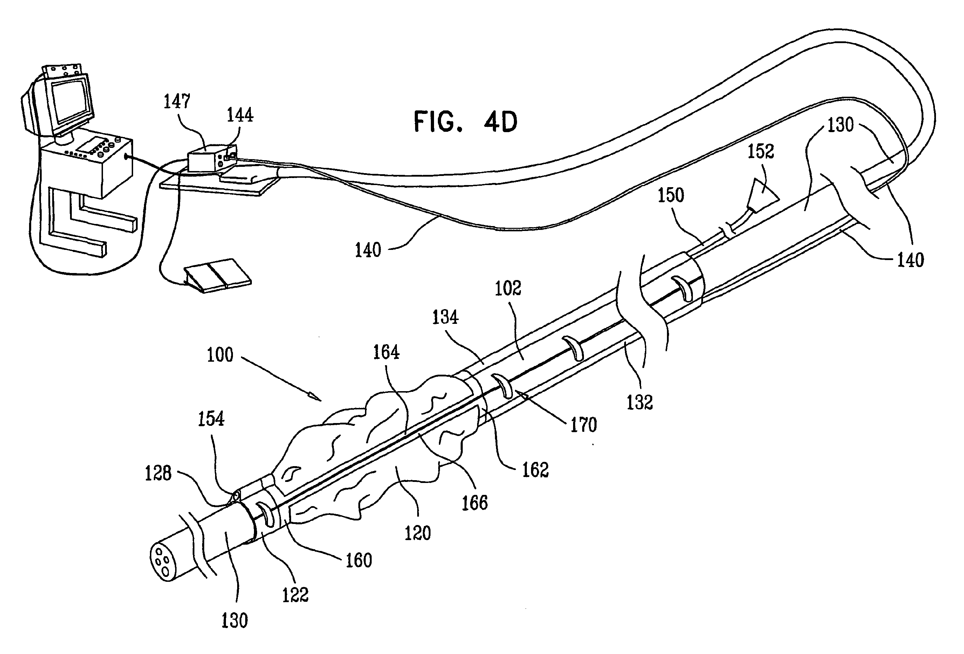

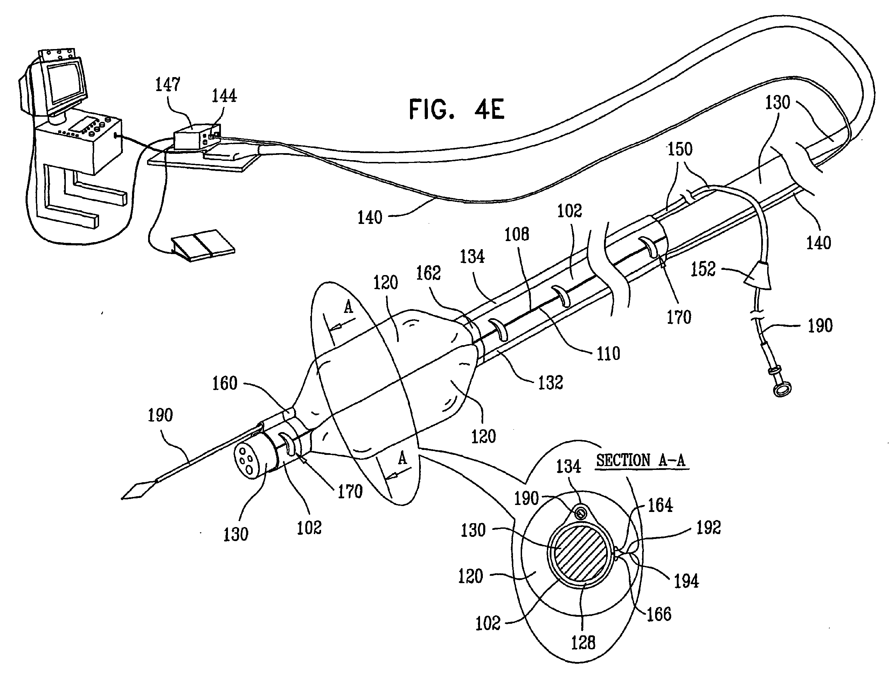

Reference is now made to Figs. 4A, 4B, 4C, 4D and 4E, which are simplified illustrations of association of the endoscope overtube of Figs. 1A - 2 with a conventional endoscope and a conventional endoscope tool.

Fig. 4A shows the overtube 100 of Figs. 1A - 2 about to be mounted onto endoscope 130. It is seen that a forward part of overtube 100 is in an expanded open orientation such that slit 106 can accommodate the thickness of endoscope 130.

Fig. 4B shows a most forward part of overtube 100, including part of balloon 120, latched in secure engagement with endoscope 130. Fig. 4C shows more of overtube 100, including all of balloon 120, latched in secure engagement with endoscope 130. Fig. 4D shows all of overtube 100, latched in secure engagement with endoscope 130. Fig. 4E illustrates the general configuration of balloon 120 when inflated and the insertion of a conventional endoscope tool 190 through the instrument channel, defined by port 152, tube 150 and second side lumen 134, which is preferably a low friction lumen comprising a flexible internal TEFLON® tube, a hydrophilic coating, or any alternative suitable low friction lumen.

Particular reference is made to Section A-A in Fig. 4E which illustrates that balloon 120, when inflated defines mutually touching facing surfaces 192 and 194 which lie generally radially outwardly from sleeve 102 between edges 164 and 166 of balloon 120.

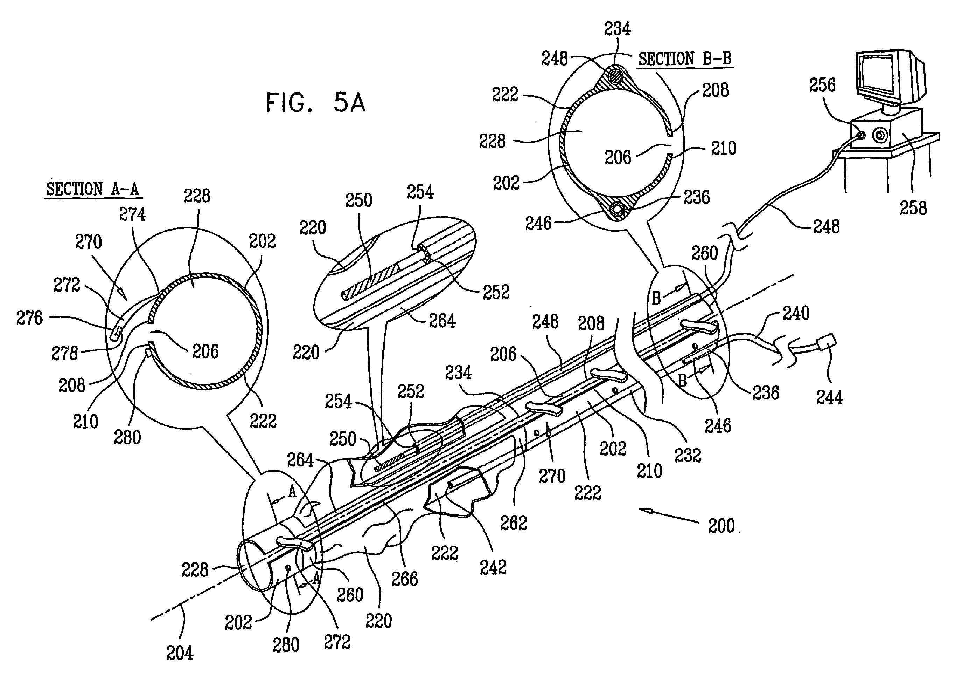

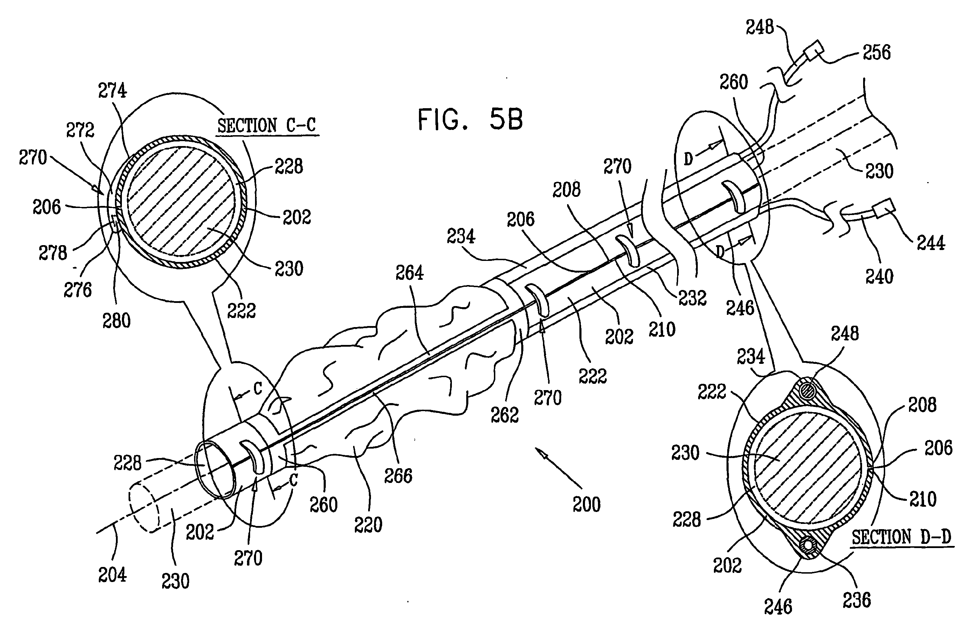

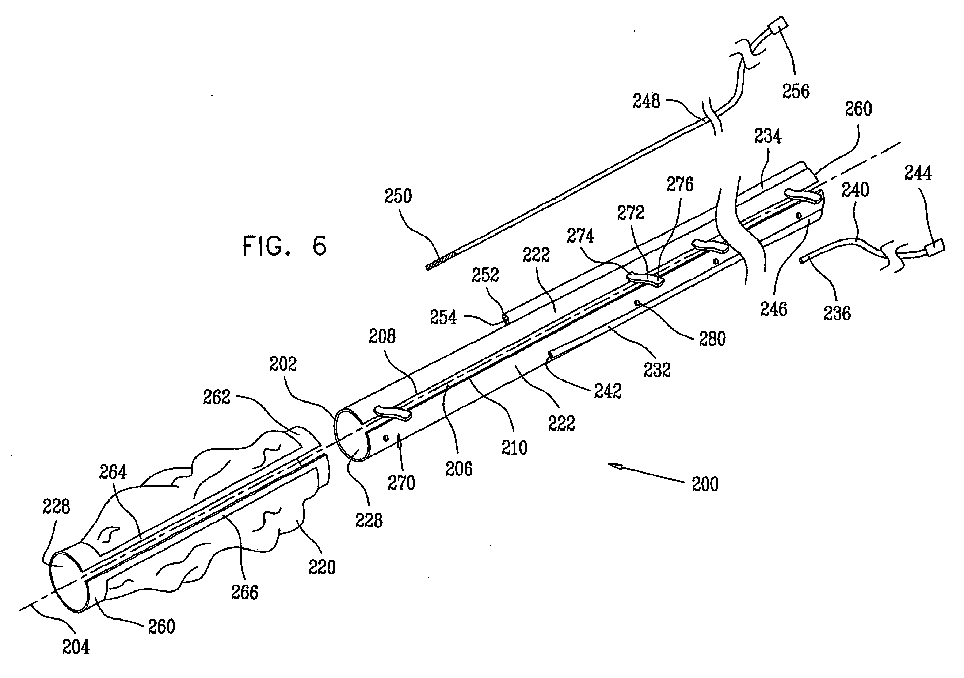

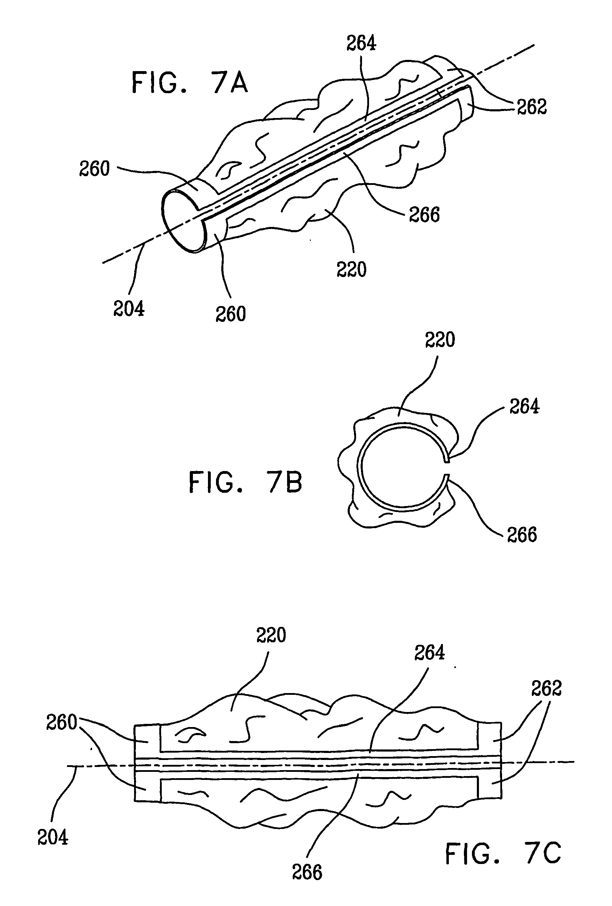

Reference is now made to Figs. 5A & 5B, which are simplified pictorial illustrations of an ultrasound overtube assembly constructed and operative in accordance with a preferred embodiment of the present invention in respective unmounted and endoscope-mounted orientations; Fig. 6, which is a simplified exploded pictorial illustration of the ultrasound overtube assembly of Fig. 5A; and Figs. 7A, 7B & 7C, which are respective simplified pictorial, end view and side view illustrations of a wrap-around balloon employed in the ultrasound overtube assembly of Figs. 5A - 6.

As seen in Figs. 5A - 6, there is provided an ultrasound overtube assembly 200, constructed and operative in accordance with a preferred embodiment of the present invention, including a generally non axially compressible, generally cylindrical tubular sleeve 202 arranged about a longitudinal axis 204. Sleeve 202 is preferably provided with an axial slit 206, thus defining axial slit edges 208 and 210 and thus is side openable and closable. Sleeve 202 is preferably resilient so as to generally circumferentially grip an endoscope onto which it is mounted.

A selectably inflatable/deflatable balloon 220, preferably a near fully circumferential wrapped balloon, is mounted over part of an outer surface 222 of sleeve 202. Preferably, the structure and mounting of balloon 220 on sleeve 202 is identical to that described hereinabove with reference to Figs. 1A - 3.

Sleeve 202 preferably has a main lumen 228 for accommodating an endoscope 230, which may be similar to endoscope 130 described hereinabove, as well as an ultrasonic medium supply and drainage lumen 232 and an ultrasonic probe accommodating lumen 234, which are circumferentially spaced from each other along outer surface 222 of sleeve 202.

Lumen 232 accommodates a forward portion 236 of an ultrasonic medium supply and drainage tube 240 and extends partially along the length of sleeve 202, outwardly of main lumen 228, to an opening 242 underlying and in fluid communication with the interior of balloon 220. Preferably, tube 240 extends from an ultrasonic medium supply and drainage connector 244 outside of sleeve 202 and the forward portion 236 thereof extends partially along and inside a rearward-facing portion 246 of lumen 232 and is fixedly and sealingly attached thereto as by a suitable adhesive, so as to provide a sealed fluid pathway communicating therewith.

Preferably, tube 240 is connected, via connector 244, to an ultrasonic medium supply and drainage system (not shown), which may be similar to balloon inflation/deflation system 147 (Figs. 4A - 4E), or to any other suitable ultrasonic medium supply and drainage mechanism, such as water injection pump or syringe.

An ultrasonic probe 248, including an ultrasonic probe head 250, extends through lumen 234, such that probe head 250 preferably lies forwardly of a forward edge 252 of lumen 234. A circumferential seal 254 seals the probe to the interior of lumen 234 rearwardly of probe head 250 and rearwardly of forward end 252 of lumen 234.

Ultrasonic probe 248 extends from an ultrasonic probe port 256 outside of sleeve 202 and is preferably fixed with respect to lumen 234. Ultrasonic probe port 256 may be connected to an ultrasound console 258. For example, ultrasound probe 248 may be a radial mini probe model PL2220B-15, and ultrasound console 258 may be HI VISION 900 system, both available from Hitachi Medical Systems GmbH, of Kreuzberger Ring 66 D-65205 Wiesbaden, Germany.

Lumen 234 preferably extends partially along the length of sleeve 202, from a rear edge 260 thereof along outer surface 222, and is preferably arranged such that forward edge 252 thereof underlies balloon 220 preferably about midway along its longitudinal extent, such that probe head 250 lies in fluid communication with the ultrasonic medium filling balloon 220.

Balloon 220 is preferably a preformed, flexible element, having a generally cylindrical configuration when assembled onto sleeve 202 and securely mounted onto endoscope 230 (Fig. 5B). Balloon 220 includes peripheral sealing surfaces which are preferably adhesively joined or heat welded onto outer surface 222 of sleeve 202. The peripheral sealing surfaces preferably include respective forward and rearward circumferential collar sealing surfaces 260 and 262 and first and second axial sealing surfaces 264 and 266 which extend parallel to slit edges 208 and 210.

It is appreciated that the generally axial slit 206 of sleeve 202 may be straight or alternatively curved, such as a straight slit parallel to longitudinal axis 204, a spiral slit along longitudinal axis 204, or a sinusoidal slit. The forward edge of sleeve 202 is preferably smooth and rounded so as to avoid damage to tissue under examination during in vivo inspection of a generally tubular body portion such as the intestine.

Preferably, sleeve 202 is shorter than endoscope 230 and is of a typical length of approximately 90 - 160 cm and has an inner diameter of approximately 10 - 14 mm and an outer diameter of approximately 12 - 16 mm, so as to be readily slidable over a conventional endoscope. Preferably, sleeve 202 is configured for mounting over endoscopes of various diameters, such as in the range of 9.5 - 13 mm. The thickness of sleeve 202 preferably is in the range of 0.3 - 2 mm, and may be constant or varying along its length.

In accordance with a preferred embodiment of the present invention, the forward portion of sleeve 202 underlying balloon 220 is relatively rigid, thereby not allowing radially inward deformation of sleeve 202 during inflation of balloon 220, so as not to inhibit slidable motion of endoscope 230 through sleeve 202 when balloon 220 is inflated. Alternatively, the forward portion of sleeve 202 underlying balloon 220 is highly flexible, thereby allowing radially inward deformation of sleeve 202 during inflation of balloon 220, so as to grip endoscope 230 and bind sleeve 202 to endoscope 230 when balloon 220 is inflated, thereby preventing sliding motion therebetween.

It is appreciated that sleeve 202 is relatively flexible, thereby being able to conform to bending of endoscope 230 onto which it is mounted, and is yet sufficiently rigid so as to allow axial displacement of the sleeve over endoscope 230 by pushing the sleeve at a rearward end thereof. Sleeve 202 may be formed of any suitable material such as silicone, PEBAX®, PVC or polyurethane. In accordance with a preferred embodiment of the present invention, the inner surface of sleeve 202 is formed of a low-friction material, such as a thin and flexible internal TEFLON® tube or a hydrophilic coating, so as to allow low resistance sliding of sleeve 202 over an endoscope when the endoscope is in a bent orientation.

It is appreciated that in accordance with a preferred embodiment of the present invention balloon 220 is generally inflatable, and can be inflated to a diameter about 3 - 10 times larger than its diameter when not inflated. In accordance with a preferred embodiment of the present invention, useful for small intestine endoscopy, the diameter of balloon 220 when fully inflated is in the range of 35 - 45 mm. Preferably, inflation of balloon 220 to a diameter less than 45 mm may be achieved using relatively low pressure, such as in the range of 10-20 millibars (1000 - 2000 Pa).

In another specific embodiment, useful for large intestine endoscopy, the diameter of balloon 220, when fully inflated, is in the range of 4 - 6 centimeters.

It is appreciated that in accordance with a preferred embodiment of the present invention, useful for in vivo inspection of a generally tubular body portion having a variable cross-sectional diameter, the expansion diameter range of balloon 220, when mounted onto sleeve 202, is larger than the maximum cross-sectional diameter of the generally tubular body portion, thereby enabling engagement of expanded balloon 220 with the interior surface of the generally tubular body portion, and anchoring of sleeve 202 thereto. Preferably, balloon 220 is a relatively soft, highly compliant balloon, operative to at least partially conform to the shape of the interior surface of the generally tubular body portion when in engagement therewith.

It is appreciated that balloon 220 may be formed of suitable well-known stretchable materials such as latex, flexible silicone, or highly flexible nylon. Balloon 220 may be compliant, semi-compliant or generally non-compliant. In the latter two cases, it may be formed of a material such as polyurethane which is less flexible, stretchable and compliant than latex, flexible silicone and highly flexible nylon. Preferably, the diameter of balloon 220 when inflated is sufficient to ensure tight anchoring in any part of the generally tubular body portion. Preferably, balloon 220 may be inflated by liquid such as water or saline. Alternatively, balloon 220 may be inflated by gas such as air or carbon dioxide.

A plurality of latches 270 preferably are provided for selectably securely mounting overtube 200 onto endoscope 230 and are distributed along the length of overtube 200 other than at locations underlying balloon 220. These latches preferably each include an arm portion 272, which is joined at one end 274 thereof, to outer surface 222 of sleeve 202 adjacent one of edges 208 and 210 of slit 206 as by adhesive or heat welding. At an opposite end 276 of each arm there is provided a first attachment portion 278 which removably engages a corresponding second attachment portion 280 mounted adjacent an opposite one of edges 208 and 210. In the illustrated embodiment, the arm portions 272 are attached adjacent edges 208 and the first attachment portion 278 is a recess which mates with a corresponding protrusion defining the second attachment portion 280. Any other suitable arrangement may be employed.

It is appreciated that overtube 200 may be selectably securely mounted onto endoscope 230 by employing any other suitable securing mechanism, such as double-sided adhesive tape or a zipper-like mechanism for joining edges 208 and 210.

Preferably, overtube 200 is mounted and secured onto endoscope 230 in a manner identical to the secure mounting of overtube 100 onto endoscope 130 as described hereinabove with reference to Figs. 4A - 4D.

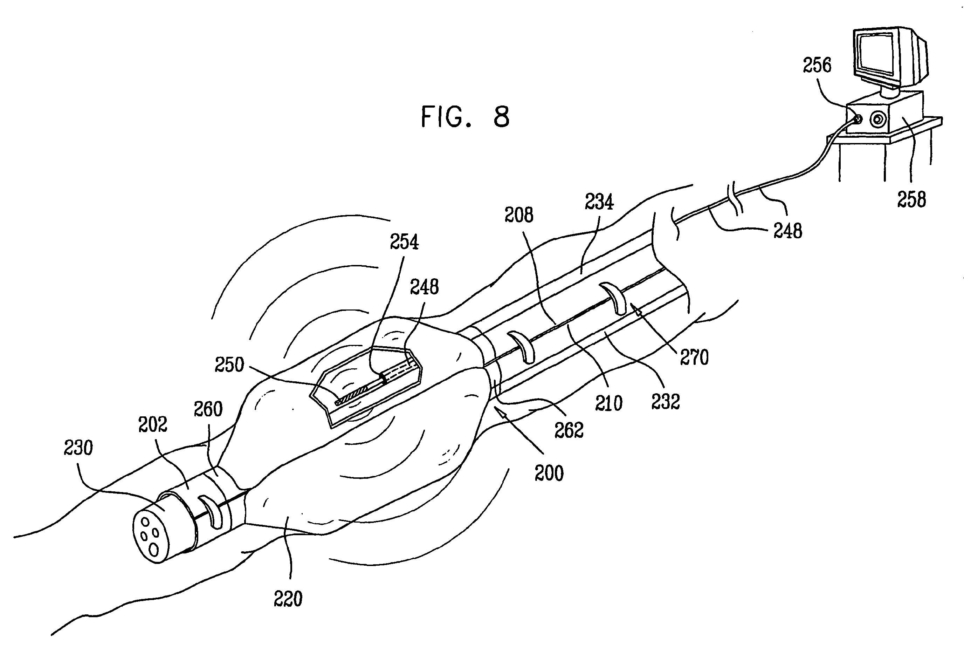

Reference is now made to Fig. 8 which is a simplified pictorial, partially cutaway, illustration of the overtube of Figs. 5A-6 in an operational orientation, associated with an endoscope and located within a tubular body portion of a patient, such as an intestine.

As seen in Fig. 8, balloon 220 is inflated by a liquid and engages the inner walls of a tubular body portion of a patient. Inflation and deflection of balloon 220 may be carried out in a manner identical to that described hereinabove with reference to Figs. 4A - 4E.

Preferably, the inflated balloon 220 is anchored to the inner walls of the tubular body portion. As further seen in Fig. 8, ultrasonic probe 248 is transmitting ultrasonic energy, as denoted by light curved lines in Fig. 8, through probe head 250. Reflected ultrasonic signals may be sensed by ultrasonic probe 248 and information therefrom may be communicated to ultrasound console 258 (Fig. 5A), which may create an ultrasound image of internal organs of the patient, as is well known in the art of ultrasound imaging.

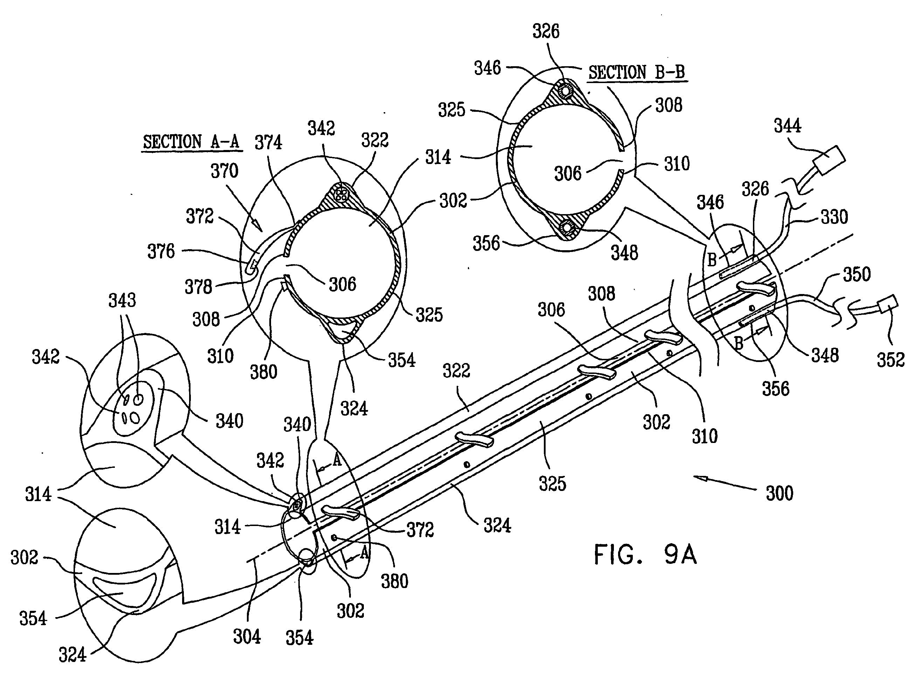

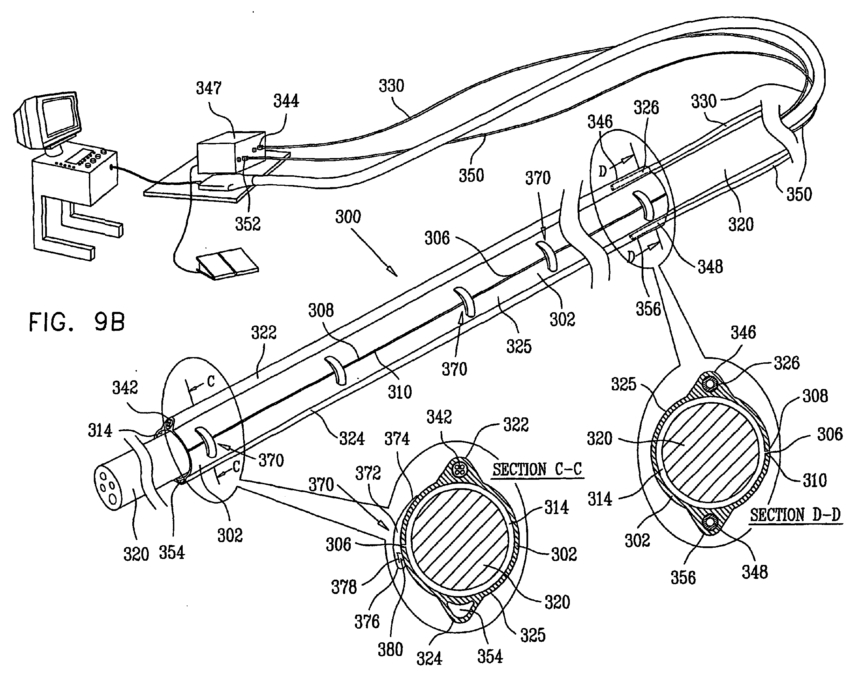

Reference is now made to Figs. 9A and 9B, which are simplified pictorial illustrations of an endoscope irrigation and suction overtube constructed and operative in accordance with a preferred embodiment of the present invention in respective open and closed orientations.

As seen in Figs. 9A and 9B, there is provided an endoscope irrigation and suction overtube 300 constructed and operative in accordance with a preferred embodiment of the present invention including a flexible, tubular, generally non axially compressible, irrigation and suction sleeve 302 arranged about a longitudinal axis 304. Irrigation and suction sleeve 302 is preferably provided with an axial slit 306, thus defining axial slit edges 308 and 310 and thus is side openable and closable. Sleeve 302 is preferably resilient so as to generally circumferentially grip an endoscope onto which it is mounted. Irrigation and suction sleeve 302 preferably has a main lumen 314 for accommodating an endoscope 320, which may be similar to endoscope 130 described hereinabove with reference to Figs. 1A- 4E, and respective irrigation and suction lumens 322 and 324, which are preferably circumferentially spaced from each other along an outer surface 325 of irrigation and suction sleeve 302.

Irrigation lumen 322 accommodates a forward portion 326 of a flexible irrigation tube 330, and preferably extends along the entire length of sleeve 302, along outer surface 325 thereof from a rear edge of sleeve 302 to a forward open end 340 thereof. An irrigation nozzle 342 having a plurality of openings 343 is preferably sealably mounted at open end 340, as by means of a suitable adhesive. Preferably, irrigation tube 330 extends from a connector 344 outside of sleeve 302 and forward portion 326 thereof extends partially along and inside a rearward-facing portion 346 of irrigation lumen 322 and is fixedly and sealingly attached thereto as by a suitable adhesive, so as to provide a sealed fluid pathway therewith.

Preferably, openings 343 of nozzle 342 are configured to generally direct irrigation fluid, such as water or saline, to one or few desired irrigation locations forward of the endoscope 320, when overtube 300 is mounted onto endoscope 320 and nozzle 342 is positioned adjacent the forward end of endoscope 320. For example, openings 343 may dispense irrigation fluid at a predetermined angle ahead of the endoscope, direct irrigation fluid aside from endoscope 320, or direct irrigation fluid to various different directions as needed. Specifically, one or few of the openings 343 of nozzle 342 may be directed towards the forward end of endoscope 320 and be utilized for irrigating and cleaning the CCD camera and optics located at the forward end of endoscope 320.

Irrigation tube 330 is preferably connected, via connector 344, to an irrigation port of an external irrigation/suction system 347, or to other suitable irrigation and/or suction mechanism, such as water injection pump or syringe.

Suction lumen 324 accommodates a forward portion 348 of a flexible suction tube 350 which extends from a connector 352 outside of sleeve 302. Suction lumen 324 extends along the entire length of sleeve 302, along outer surface 325 thereof from a rear edge of irrigation and suction sleeve 302 to an open end 354. Suction tube 350 extends from port 352, partially along and inside a rearward portion 356 of suction lumen 324 and is fixedly attached thereto as by a suitable adhesive.

Suction tube 350 is preferably connected, via connector 352, to a suction port of external irrigation/suction system 347, or to other suitable suction and/or irrigation mechanism, such as suction pump or syringe.

It is appreciated that the generally axial slit 306 of irrigation and suction sleeve 302 may be straight or curved, such as a straight slit parallel to longitudinal axis 304, a spiral slit along longitudinal axis 304, or a sinusoidal slit.

Preferably, irrigation and suction sleeve 302 is shorter than endoscope 320. Sleeve 302 typically has a length of approximately 90 - 160 cm, an inner diameter of approximately 10 - 14 mm and an outer diameter of approximately 12 - 16 mm, so as to be readily slidable over a conventional endoscope. Preferably, sleeve 302 is configured for mounting over endoscopes of various diameters, such as in the range of 9.5 - 13 mm. The thickness of sleeve 302 is preferably in the range of 0.3 - 2 mm, and may be constant or varying along its length.

It is appreciated that sleeve 302 is relatively flexible, thereby being able to conform to bending of endoscope 320 onto which it is slidably mounted, and is yet sufficiently rigid so as to allow sliding over endoscope 320 by pushing it forward from a rearward end thereof. Irrigation and suction sleeve 302 may be formed of any suitable material such as silicone, PEBAX®, PVC or polyurethane. In accordance with a preferred embodiment of the present invention, the inner surface of sleeve 302 is formed of a low-friction material, such as a thin and flexible internal TEFLON® tube or a hydrophilic coating, so as to allow low resistance sliding of sleeve 302 over an endoscope in a bent orientation.

A plurality of latches 370 are provided for selectably securely mounting irrigation and suction overtube 300 onto endoscope 320 and are distributed along the length of overtube 300. These latches preferably each include an arm portion 372, which is joined at one end 374 thereof, to outer surface 325 of sleeve 302 adjacent one of edges 308 and 310 of slit 306 as by adhesive or heat welding. At an opposite end 376 of each arm there is provided a first attachment portion 378 which removably engages a corresponding second attachment portion 380 mounted adjacent an opposite one of edges 308 and 310. In the illustrated embodiment, the arm portions 372 are attached adjacent edges 308 and the first attachment portion 378 is a recess which mates with a corresponding protrusion defining the second attachment portion 380. Any other suitable arrangement may be employed.

Irrigation and suction overtube 300 may be side-mounted on endoscope 320 identically to the mounting of overtube 100 onto endoscope 130 as described hereinabove with reference to Figs. 4A - 4D.

In accordance with a preferred embodiment of the present invention which is suitable for in vivo inspection and treatment of a tubular body portion such as the intestine, pressurized fluid from system 347, such as water or saline, is applied to the internal walls of the tubular body portion forwardly of endoscope 320, thereby irrigating the internal surface of the inspected tubular body portion and removing remains such as food or feces. Such irrigation may be used during endoscopy examination for clearing an intestinal surface to be inspected or treated, for cleaning a diverticulum or another pathology, or for any other suitable application.

Alternatively or additionally, other fluids may be applied, such as an antiseptic fluid, a medication, a cover layer for protecting an internal surface of the tubular body portion, or any other suitable fluid. It is appreciated that multiple fluids may be applied, alternately or simultaneously, using the apparatus of the present invention. It is appreciated that more than one irrigation lumen 322 can be provided.

In accordance with a preferred embodiment of the present invention which is suitable for in vivo inspection and treatment of a tubular body portion such as the intestine, suctioning is performed in the inner volume of the tubular body portion forwardly of endoscope 320, thereby removing irrigation fluid and remains such as food or feces from the inspected portion of the tubular body portion.

It is appreciated that irrigation and suction overtube 300 and endoscope 320 may be operated jointly as an intestinal examination enhancement system having real-time preparation deficiency amelioration functionality. Such an intestinal examination enhancement may preferably be performed by optically sensing a preparation deficiency in the intestine by endoscope 320, thereafter side-mounting overtube 300 onto endoscope 320 and sliding overtube 300 along the endoscope 320 into the sensed preparation deficiency location in the intestine, and subsequently ameliorating the preparation deficiency by flushing the preparation deficiency location with liquid through overtube 300.

In accordance with a preferred embodiment of the present invention, irrigation liquid supply through irrigation lumen 322 and liquid evacuation by suctioning through suction lumen 324 are performed simultaneously by irrigation/suction system 347.

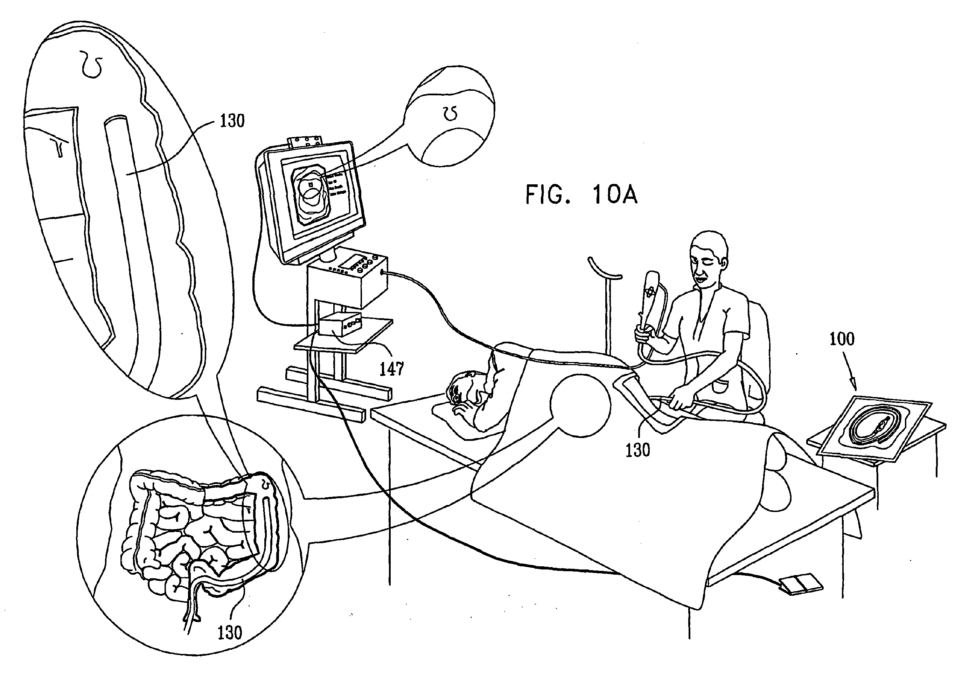

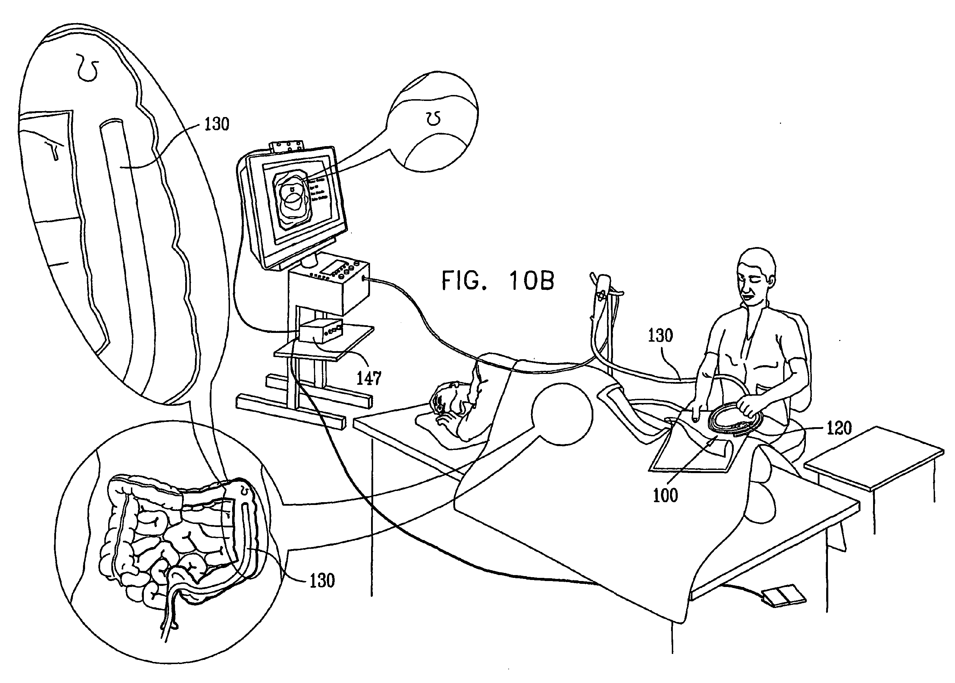

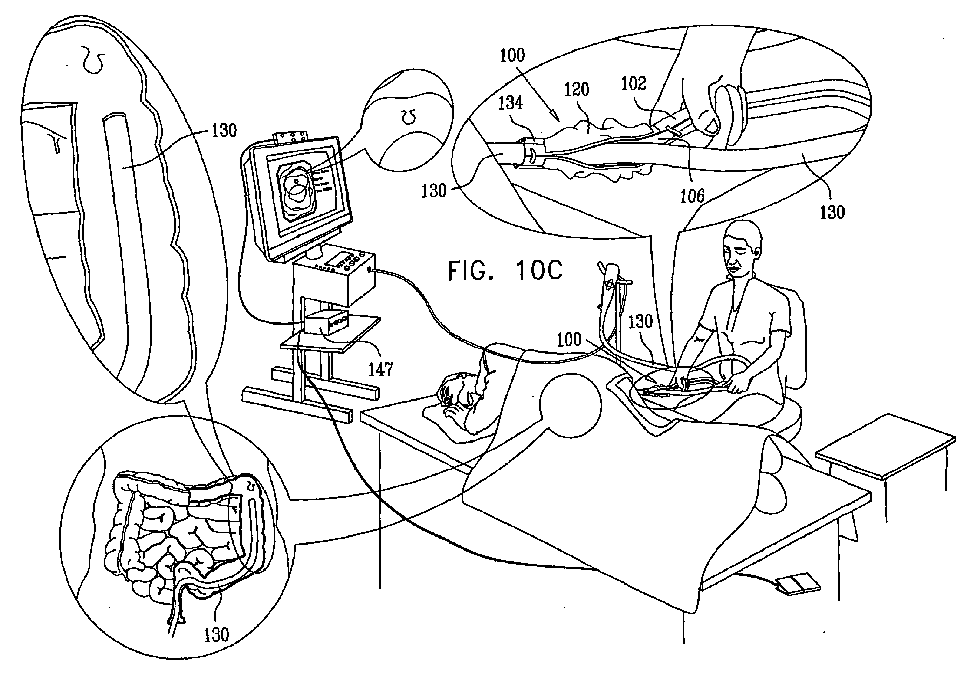

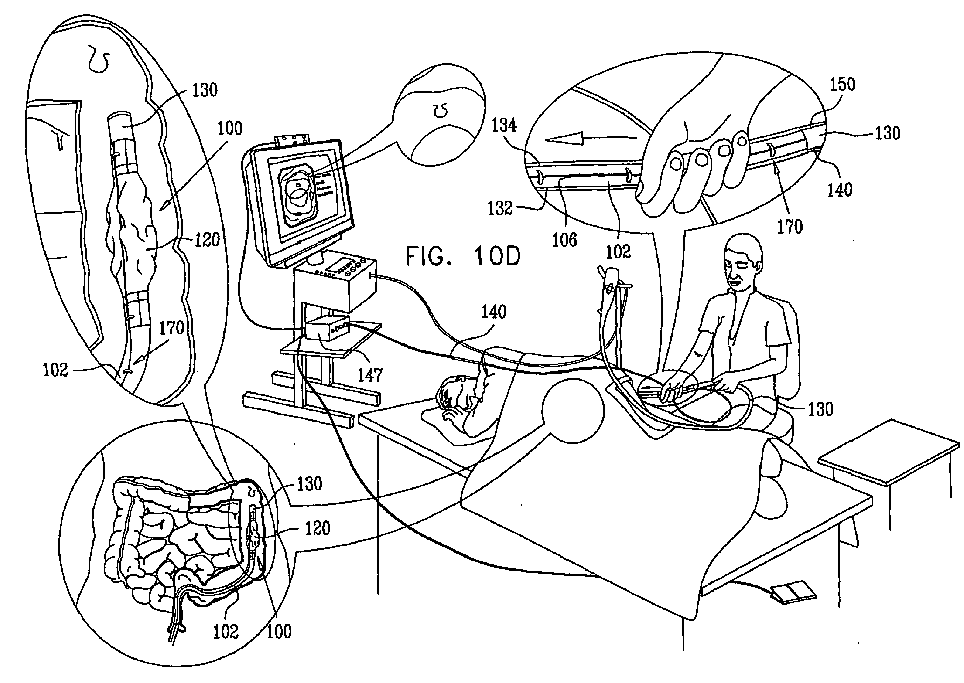

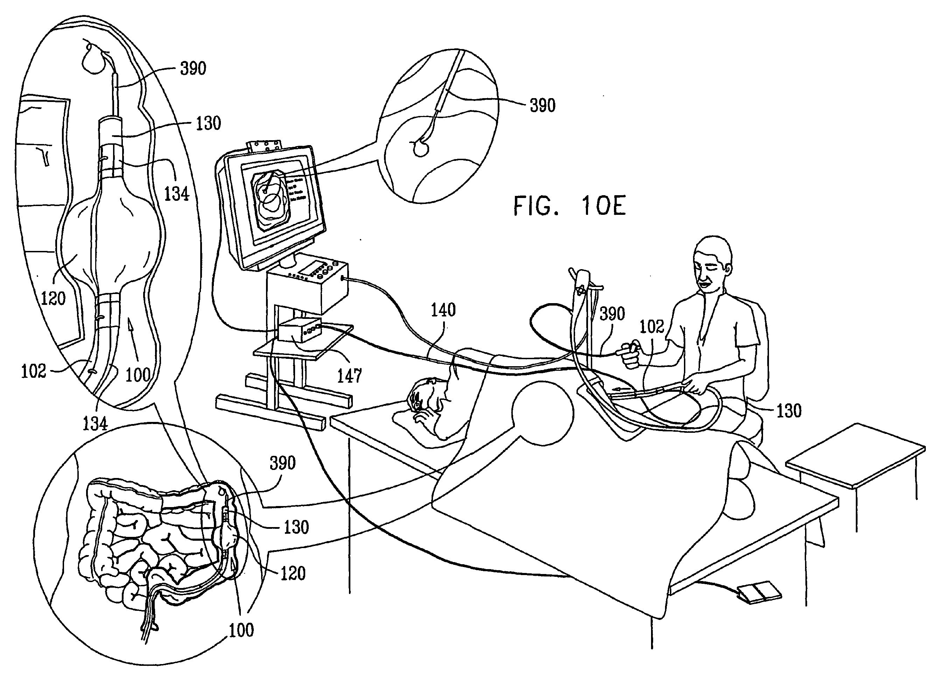

Reference is now made to Figs. 10A, 10B, 10C, 10D and 10E, which are simplified pictorial illustrations of use of the overtube of the embodiment of Figs. A - 4E in an endoscopic treatment. Fig. 10A shows initial visual detection of a polyp in the colon of a patient by use of a conventional endoscope system including an endoscope, such as endoscope 130 (Figs. 1B & 4A - 4E) and a display screen. An endoscope overtube, such as endoscope overtube 100, described hereinabove with reference to Figs. 1 - 4E, constructed and operative in accordance with a preferred embodiment of the present invention is available for use as needed. Preferably it is kept in a sealed package.

As seen in Fig. 10B, once the operator detects the polyp as seen on the display screen and decides that treatment is required, the operator opens the sealed package and accesses the overtube 100. It is noted that should no treatment be required in the course of the conventional endoscopic examination, the overtube 100 need not be used and can be retained in its sealed package for future use as needed.

Fig. 10C shows an initial stage in side mounting of the endoscope overtube 100 onto endoscope 130. The mounting procedure may be substantially identical to that described hereinabove with reference to Figs. 4A - 4D.

Fig. 10D illustrates forward sliding displacement of the overtube 100 along endoscope 130 such that the overtube 100 is positioned within the colon of the patient adjacent a forward end of endoscope 130.

Fig. 10E shows inflation of wrapped balloon 120 of overtube 100 to circumferential engagement with the colon, thereby to securely position the overtube 100 and thus the endoscope 130 in the colon and removal of the polyp, using a conventional polyp removal tool 390. Preferably, the polyp removal tool 390 passes through an instrument channel of the endoscope 130. Alternatively, it may pass through a suitable lumen of the overtube 100, such as second side lumen 134 of overtube 100, describe hereinabove with reference to Figs. 1A - 4E.

It is appreciated that various other endoscope tools may be employed as needed alternatively or additionally to polyp removal tool 390. It is appreciated that the general methodology described hereinabove with reference to Figs. 10A - 10E is applicable also to the embodiment of Figs. 5A - 8 described hereinabove. It is appreciated that various

It is appreciated that various other conditions requiring a follow-on endoscopic function employing overtube 100 may be detected by endoscope 130, such as a bleeding point, a plurality of polyps and a stricture.

It is further appreciated that the provision of a wrapped balloon on a side-mountable overtube enables various overtube structures and functionalities to be realized in a real-time, just-as-needed methodology as described hereinabove with reference to Figs. 10A - 10E, wherein the overtube need not be employed until after insertion of the endoscope in the patient and determination by the endoscope operator, typically by visual examination using the endoscope, that use of a balloon-bearing overtube is called for.

In accordance with a preferred embodiment of the present invention, wrapped balloon 120 is an anchoring balloon which anchors overtube 100 to the interior of the intestine of the patient when inflated, at the balloon inflation location. Preferably, sliding motion is caused by the operator following the anchoring of balloon 120 to the intestine, to advance or retract endoscope 120 in the intestine, as well known in the art of push-pull endoscopy, and particularly in the art of single balloon endoscopy.

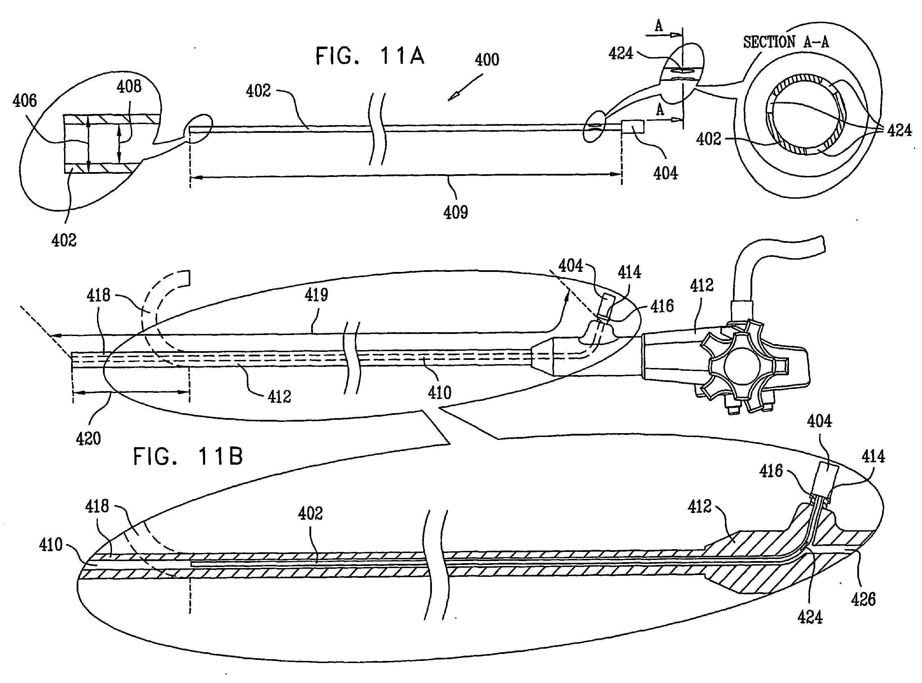

Reference is now made to Fig. 11A, which is a simplified pictorial illustration of a hollow-tube stiffener constructed and operative in accordance with a preferred embodiment of the present invention and to Fig. 11B, which is a simplified pictorial illustration of the stiffener of Fig. 11A fully inserted in an instrument channel of a conventional endoscope.

As seen in Fig. 11A, there is provided a stiffener 400 which preferably comprises a hollow tube 402 having a cap 404 fixedly mounted on one end thereof. Cap 404 may assume any suitable shape and may comprise a handle for holding stiffener 400 while inserting it into the instrument channel of the endoscope. In accordance with one embodiment of the present invention, the hollow tube 402 is formed of a polymer such as PVC, TEFLON®, PEBAX® having a preferred outer diameter 406 of 2.3 - 4.0 mm and an inner diameter 408 of 1.0 - 2.5 mm. In accordance with another embodiment of the invention, the hollow tube is formed of a metal such as stainless steel or nitinol, having a preferred outer diameter of 2.5 - 3.5 mm and an inner diameter of 1.2 - 2.5 mm.

The length 409 of the hollow tube 402 forward of the cap 404 is preferably selected to be such that it extends up to but not into the bending portion at the forward end of the endoscope. For example, when an endoscope such as a Pentax gastroscope model EG-2930K, having a working length of 105cm including a forward bending portion of length of approximately 7cm is employed, the length of the hollow tube 402 forward of the cap 404 is preferably 113cm. As another example, when an endoscope such as an Olympus GIF-1T100, having a working length of 103cm including a forward bending portion of length of approximately 7cm is employed, the length of the hollow tube 402 forward of the cap 404 is preferably 111cm.

In accordance with an embodiment of the present invention, the forward end of the hollow tube 402 or any other elongate stiffener element, when the elongate stiffener element is fully inserted into the instrument channel of an endoscope, does not extend into the forward bending portion of the endoscope to an extent which appreciably limits the bendability of the bending portion of the endoscope. For example, when an endoscope such as a Pentax gastroscope model EG-2930K, having a working length of 105cm including a forward bending portion of length of approximately 7cm is employed, the length of the hollow tube 402 forward of the cap 404 is preferably less than 116cam.

Fig. 11B shows the stiffener 400 fully inserted into an instrument channel 410 of an endoscope 412, wherein the forward end of cap 404 lies against a facing wall 414 of an instrument channel port 416. It is seen that the forward end of the stiffener 400 terminates just rearward of the bending portion 418, inasmuch as the length 409 of the hollow tube 402 forward of cap 404 is approximately equal to the length 419 of the instrument channel 410 less the length 420 of the bending portion 418.

In accordance with a preferred embodiment of the present invention, the hollow tube 402 is formed with suction holes 424 distributed about the circumference of tube 402 at a location forward of but adjacent to cap 404, communicating with a conventional endoscope suction channel 426. Preferably suction holes 424 are somewhat elongated so as to accommodate slight variations in the construction of various endoscopes. It is a particular feature of the present invention that suctioning may be carried out through the stiffener by virtue of the provision of hollow tube 402 and suction holes 424.

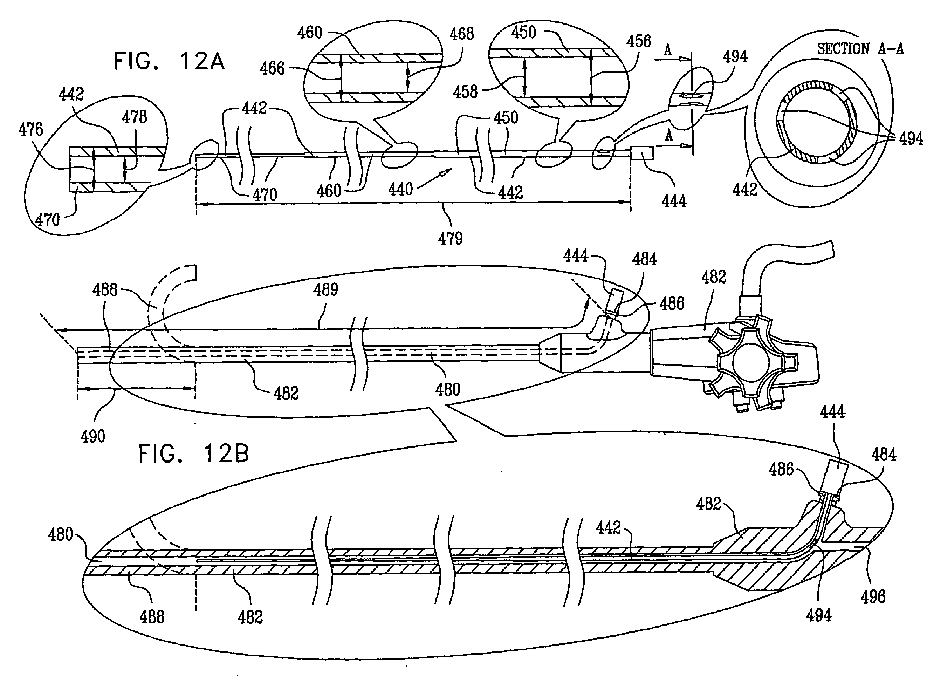

Reference is now made to Fig. 12A, which is a simplified pictorial illustration of a stiffener having non uniform stiffness along its length, constructed and operative in accordance with a preferred embodiment of the present invention and to Fig. 12B, which is a simplified pictorial illustration of the stiffener of Fig. 12A fully inserted in a conventional endoscope.

As seen in Fig. 12A, there is provided a stiffener 440 which preferably, but not necessarily, comprises a hollow tube 442 having a cap 444 fixedly mounted on one end thereof. It is a particular feature of this embodiment of the present invention that the stiffener 440 has differing stiffness at different locations along its length. This may be realized in various ways, Such as by varying the materials, thickness or inner diameter of tube 442 or of a solid stiffener. In accordance with one embodiment of the invention, the hollow tube 442 is formed of a polymer such as PVC, TEFLON®, PEBAX® having at a first length portion thereof 450 an outer diameter 456 of 3.5mm and an inner diameter 458 of 1.4mm, at a second length portion thereof 460 an outer diameter 466 of 3.0mm and an inner diameter 468 of 1.7mm and at a third length portion thereof 470 an outer diameter 476 of 2.7mm and an inner diameter 478 of 2.0mm. It is appreciated that the ordering of the various extents of stiffness along the length of the stiffener may depend on the application. Variation in stiffness may be stepwise or generally continuous. In accordance with another embodiment of the invention, the hollow tube is formed of a metal such as stainless steel or nitinol.

The length 479 of the hollow tube 442 forward of the cap 444 is preferably selected to be such that it extends up to but not into the bending portion at the forward end of the endoscope as described hereinabove with reference to Figs. 11A & 11B.

Fig. 12B shows the stiffener 440 fully inserted into an instrument channel 480 of an endoscope 482, wherein the forward end of cap 444 lies against a facing wall 484 of an instrument channel port 486. It is seen that the forward end of the stiffener 440 terminates just rearward of the bending portion 488, inasmuch as the length 479 of the hollow tube 442 forward of cap 444 is approximately equal to the length 489 of the instrument channel 480 less the length 490 of the bending portion 488.

Preferably, the hollow tube 442 is formed with suction holes 494 distributed about the circumference of tube 442 at a location forward of but adjacent to cap 444, communicating with a conventional endoscope suction channel 496. Thus suctioning may be carried out through the stiffener 440 by virtue of the provision of hollow tube 442 and suction holes 494.

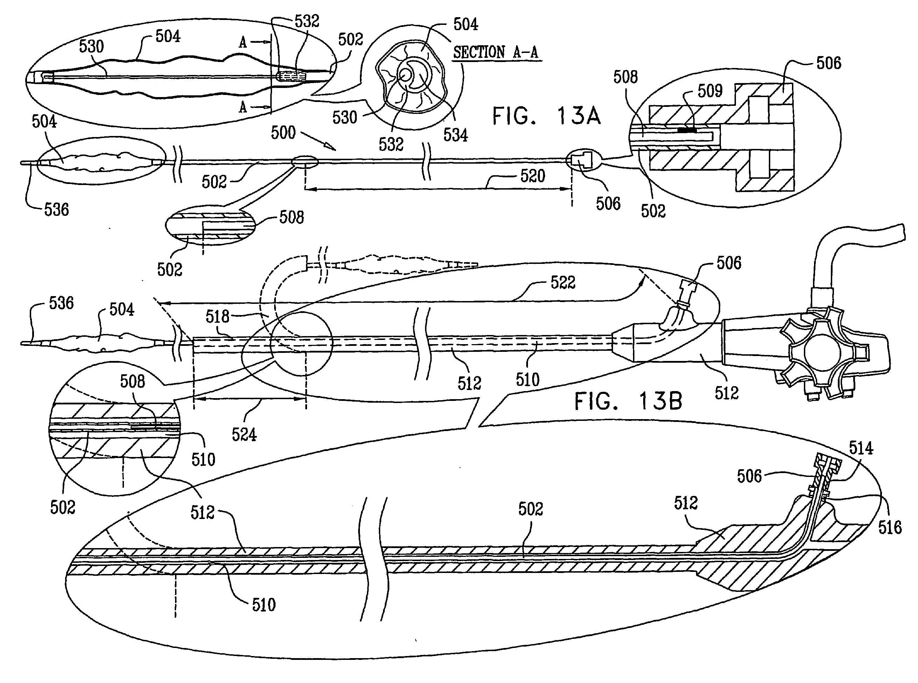

Reference is now made to Fig. 13A, which is a simplified pictorial illustration of a balloon catheter having non-uniform stiffness along its length, constructed and operative in accordance with a preferred embodiment of the present invention and to Fig. 13B, which is a simplified pictorial illustration of the balloon catheter of Fig. 13A fully inserted in a conventional endoscope.

As seen in Fig. 13A, there is provided a balloon catheter 500 which comprises a hollow tube 502, adjacent a forward end of which is mounted a balloon 504. A connector 506 is fixedly mounted on a rearward end of hollow tube 502. It is a particular feature of this embodiment of the present invention that the balloon catheter 500 has differing stiffness at different locations along its length rearwardly of balloon 504. In the embodiment of Figs. 13A & 13B, this is realized by providing a stiffener 508 which extends partially but not entirely along the length of hollow tube 502. Preferably stiffener 508 is fixed at a rearward end thereof to connector 506, as by an adhesive 509, in a manner which permits fluid communication between connector 506 and tube 502. Stiffener 508 preferably extends up to but not into the bending portion at the forward end of an endoscope through an instrument channel of which the balloon catheter extends.

For example, when an endoscope such as a Pentax gastroscope model EG-3430K, having a working length of 105cm including a forward bending portion of length of approximately 9cm is employed, the length of the stiffener 508 forward of connector 506 is preferably approximately 110cm. As another example, when an endoscope such as a Olympus gastroscope model GIF-1T100, having a working length of 103cm including a forward bending portion of length of approximately 7cm is employed, the length of the stiffener 508 forward of the connector 506 is preferably 111cm. Stiffener 508 is preferably formed of a metal such as stainless steel or nitinol.

Fig. 13B shows the balloon catheter 500 fully inserted into an instrument channel 510 of an endoscope 512, wherein the forward end of connector 506 lies against a facing wall 514 of an instrument channel port 516. It is seen that the forward end of the stiffener 508 terminates just rearward of the bending portion 518 of the endoscope 512, inasmuch as the length 520 of the stiffener 508 forward of connector 506 is approximately equal to the length 522 of the instrument channel 510 less the length 524 of the bending portion 518.

In the illustrated embodiment, balloon 504 is mounted on a balloon support wire 530 which is mounted via a mounting cap 532 onto a forward end of hollow tube 502. Mounting cap 532 also comprises an inflation/deflation conduit 534 which communicates with the interior of balloon 504 and with the interior of hollow tube 502 for providing desired inflation and deflation of balloon 504. A forward tip 536 is mounted on a forward end of balloon support wire 530. Respective rearward and forward ends of balloon 504 preferably are sealingly secured to both the forward end of hollow tube 502 at mounting cap 532 and to forward tip 536.

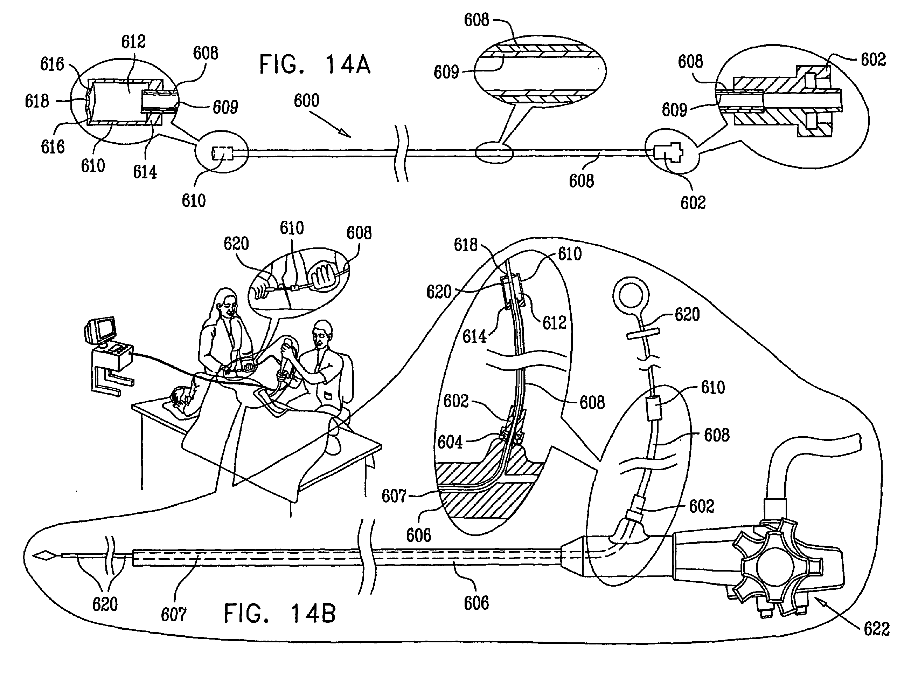

Reference is now made to Fig. 14A, which is a simplified partially sectional illustration of an instrument channel extender constructed and operative in accordance with a preferred embodiment of the present invention and to Fig. 14B, which is a simplified partially sectional, partially pictorial illustration of the use of the instrument channel extender of Fig. 14A with a conventional endoscope in accordance with a preferred embodiment of the present invention.

As seen in Figs. 14A & 14B, there is provided an instrument channel extender 600 including an instrument channel port connector 602, which is configured for removable connection to a conventional instrument channel port, such as port 604, of a conventional endoscope, such as endoscope 606 having an instrument channel 607. Connector 602 is preferably a luer-type connector which is sealingly connected to an instrument channel extension tube 608, such as a flexible tube formed of a suitable material, such as silicon, PEBAX®, PVC, TEFLON® and polyurethane.

Preferably, the inner surface of instrument channel extension tube 608 displays low friction characteristics. The low friction characteristics of the inner surface may be inherent in the material from which the instrument channel extension tube 608 is formed or may be provided by addition of a coating, layer or other material. In accordance with a preferred embodiment of the present invention, there is provided a low friction inner surface layer 609 along the inner surface of instrument channel extension tube 608. For example, layer 609 may comprise a hydrophilic coating such as 3-TS-12, possibly used in conjunction with primer 2-TS-96 both available from Hydromer Inc. of HYDROMER, INC. 35 Industrial Parkway Branchburg, NJ 08876 ,USA.

Preferably instrument channel extension tube 608 has an inner diameter in the range of 3 - 5 mm, typical of conventional endoscope instrument channels, and a corresponding outer diameter in the range of 4 - 7 mm. Most preferred dimensions are inner diameter 4.2 - 5.0 mm, outer diameter 5.5 - 6.0 mm. Instrument channel extension tube 608 preferably has a length in the range of 15 - 80 cm and most preferably in the range of 20 - 40 cm.

Preferably a remote instrument channel type port 610 is sealingly fixed to an end of instrument channel extension tube 608 opposite from connector 602. Alternatively port 610 may be obviated. Port 610 preferably includes a generally cylindrical housing 612 having a tube connection portion 614 at a first end thereof and has a flexible apertured portion 616 at a second end thereof which is configured to permit stretching and thus expansion of an aperture 618 formed therein.

Fig. 14B depicts an operational setting, where a catheter 620 is being inserted through instrument channel 607 of endoscope 606 via the instrument channel extender 600 by a catheter operator and the endoscope 606 is being operated by an endoscope operator. Connector 602 is connected to port 604 of endoscope 606 and instrument channel extension tube 608 extends away from the operator controls 622 of the endoscope 606, thereby allowing the endoscope operator and the catheter operator to work side by side with sufficient space therebetween so as to enable them to work comfortably and efficiently.

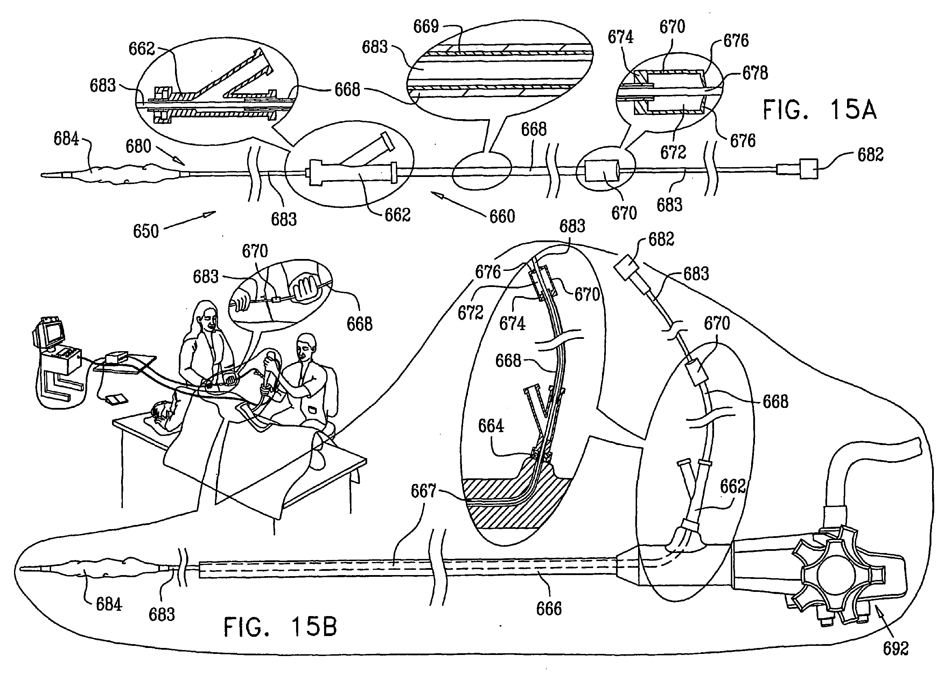

Reference is now made to Fig. 15A, which is a simplified partially sectional illustration of a catheter assembly incorporating an instrument channel extender, constructed and operative in accordance with a preferred embodiment of the present invention and to Fig. 15B, which is a simplified partially sectional, partially pictorial illustration of the use of the catheter assembly of Fig. 15A with a conventional endoscope in accordance with a preferred embodiment of the present invention.

As seen in Figs. 15A & 15B, there is provided a catheter assembly 650 incorporating an instrument channel extender 660, which is similar to instrument channel extender 600 but preferably includes a different type of instrument channel port connector 662, preferably in the form of a Y-connector, which is configured for removable connection to a conventional instrument channel port, such as port 664, of a conventional endoscope, such as endoscope 666 having an instrument channel 667. Connector 662 is preferably a luer-type connector which is sealingly connected to an instrument channel extension tube 668, such as a flexible tube formed of a suitable material, such as silicon, PEBAX®, PVC, TEFLON® and polyurethane.

Preferably, the inner surface of instrument channel extension tube 668 displays low friction characteristics. The low friction characteristics of the inner surface may be inherent in the material from which the instrument channel extension tube 668 is formed or may be provided by addition of a coating, layer or other material. In accordance with a preferred embodiment of the present invention, there is provided a low friction inner surface layer 669 along the inner surface of instrument channel extension tube 668. For example, layer 669 may comprise a hydrophilic coating such as 3-TS-12, possibly used in conjunction with primer 2-TS-96 both available from Hydromer Inc. of HYDROMER, INC. 35 Industrial Parkway Branchburg, NJ 08876 ,USA.

Preferably instrument channel extension tube 668 has an inner diameter in the range of 3 - 5 mm, typical of conventional endoscope instrument channels, and a corresponding outer diameter in the range of 4 - 7 mm. Most preferred dimensions are inner diameter 4.2 - 5.0 mm, outer diameter 5.5 - 6.0 mm. Instrument channel extension tube 668 preferably has a length in the range of 15 - 80 cm and most preferably in the range of 20 - 40 cm.

Preferably a remote instrument channel type port 670 is sealingly fixed to an end of instrument channel extension tube 668 opposite from connector 662. Alternatively port 670 may be obviated. Port 670 preferably includes a generally cylindrical housing 672 having a tube connection portion 674 at a first end thereof and has a flexible apertured portion 676 at a second end thereof which is configured to permit stretching and thus expansion of an aperture 678 formed therein.

A catheter, preferably a balloon catheter 680, is shown extending through instrument channel extender 660 and includes a balloon catheter connector 682 at a rearward end of a catheter tube 683, and a selectably inflatable balloon 684 at a forward portion of tube 683.

Fig. 15B depicts an operational setting, where catheter 680 is being inserted through instrument channel 667 of endoscope 666 via the instrument channel extender 660 by a catheter operator and the endoscope 666 is being operated by an endoscope operator. Connector 662 is connected to port 664 of endoscope 666 and instrument channel extension tube 668 extends away from the operator controls 692 of the endoscope 666, thereby allowing the endoscope operator and the catheter operator to work side by side with sufficient space therebetween so as to enable them to work comfortably and efficiently.

It will be appreciated by persons skilled in the art that the present invention is not limited by what has been particularly shown and described herein above. Rather the scope of the present invention includes both combinations and subcombinations of the various features described hereinabove as well as variations and modifications which would occur to persons skilled in the art upon reading the specifications and which are not in the prior art.

## Claims

1. A side-mountable overtube (100) for use with an endoscope (130), said overtube (100) comprising:
a sleeve (102) formed with a slit (106), extending entirely along its length, and with a balloon inflation/deflation lumen (132) communicating with a balloon opening (142) at a balloon opening location intermediate along its length; and
a sclectably inflatable/deflatable balloon (120) mounted onto said sleeve (102) in a manner such that it does not extend entirely across said slit (106) and thereby is side mountable along with said sleeve (102) onto an endoscope (130), an interior of said balloon (120) communicating with said balloon opening (142) at said balloon opening location for selectable inflation/deflation of said balloon (120).

2. A side-mountable overtube (100) according to claim I and wherein said inflatable/deflatable balloon (120), when inflated, defines mutually touching inflated balloon surfaces (192, 194) extending generally along edges (108, 110) of said slit (106).

3. A side-mountable overtube (100) according to either of claims 1 and 2 and wherein said overtube (100) is configured for removable attachment to said endoscope (130) when said balloon (120) is inflated, thereby preventing sliding motion between said overtube (100) and said endoscope (130).

4. A side-mountable overtube (100) according to either of claims 1 and 2 and wherein said overtube (100) is configured to permit relative sliding motion between said overtube (100) and said endoscope (130) when said balloon (120} is inflated.

5. A side-mountable overtube (100) according to any of claims 1 - 4 and wherein said overtube inflatable/deflatable balloon (120) comprises at least first and second axial sealing surfaces (164, 166) which extend generally parallel to said slit (106).

6. A side-mountable overtube (100) according to any of claims 1 - 5 and also comprising a side lumen (134) extending at least partially along the length of said sleeve (102) and traversing said balloon (120).

7. A side-mountable overtube (100) according to claim 6 and also comprising an instrument channel tube (150) communicating with a rearward portion of said side lumen (134) and an instrument channel port (152) which communicates with an end portion of said instrument channel tube (150).

8. A side-mountable overtube (100) according to any of claims 1 - 7 and also comprising a securing mechanism (170) for selectably securing said side-mountable overtube (100) on said endoscope (130).

9. A side-mountable overtube (100) according to claim 8 and wherein said securing mechanism (170) comprises a double-sided adhesive tape adapted to selectably join opposing axial edges (108, 110) of said slit (106).

10. A side-mountable overtube (100) according to any of ctaims 1 - 9 and also comprising an inflation/deflation functionality (147) operative for selectable inflation and deflation of said balloon (120).

## Patentansprüche

1. Ein seitlich-montierbares Überrohr (100) zur Anwendung mit einem Endoskop (130), wobei das Überrohr (100) umfaßt;
eine Hülse (102), die mit einem sich ihre Länge entlang erstreckenden Schlitz (106) und mit einem Ballon-Inflation/ Deflation-Lumen (132) gebildet ist, das mit einer Ballon-Öffnung (142) am Ort der Ballon-Öffnung, der sich in der Mitte ihrer Länge befindet, in Verbindung steht; und
ein auswählbar auf-/ ausblasbares Ballon (120), das auf der genannten Hülse (120) angeordnet ist, so dass es sich nicht völlig über den genannten Schlitz (106) erstreckt und wobei es mit der Hülse zusammen auf ein Endoskop (130) seitlich montierbar ist, wobei ein Innenbereich des Ballons (120) mit der Ballon-Öffnung (142) am genannten Ort der Ballon-Öffnung zur auswählbarer Inflation/Deflation des Ballons (120) verbunden ist.

2. Ein seitlich montierbares Überrohr (100) nach Anspruch 1, wobei das erwähnte auf-/ausblasbares Ballon (120), wenn es aufgeblasen ist, gegenseitig anrührende, aufgeblasene Ballonoberflächen (192, 194) definiert, die sich hauptsächlich entlang der Kanten (108, 110) des Schlitzes (106) erstrecken.

3. Ein seitlich montierbares Überrohr (100) nach einem der Ansprüche 1 und 2, wobei das Überrohr (100) zur ablösbarer Befestigung an dem erwähnten Endoskop (130) ausgelegt ist, wenn das Ballon (120) aufgeblasen ist, wodurch es eine Gleitbewegung zwischen dem genannten Überrohr (100) und dem Endoskop (130) verhindert.

4. Ein seitlich montierbares Überrohr (100) nach einem der Ansprüche 1 und 2, wobei das Überrohr (100) ausgelegt ist, um eine relative Gleitbewegung zwischen dem genannten Überrohr (100) und dem erwähnten Endoskop (130) zu zulassen, wenn das Ballon (120) aufgeblasen ist.

5. Ein seitlich montierbares Überrohr (100) nach einem der Ansprüche 1 - 4, wobei das erwähnte Überrohr auf-/ausblasbares Ballon (120) zumindest erste und zweite axiale Dichtungsoberflächen (164, 166) umfasst, welche sich hauptsächlich parallel zum erwähnten Schlitz (106) erstrecken.

6. Ein seitlich montierbares Überrohr (100) nach einem der Ansprüche 1 - 5, umfassend auch ein zumindest teilweise entlang der Länge der erwähnten Hülse (102) verlaufendes und das Ballon (120) überquerendes Lumen (134).

7. Ein seitlich montierbares Überrohr (100) nach Anspruch 6, umfassend auch ein Instrumenten-Kanal-Rohr ( 150), das mit einem hinteren Teil des erwähnten Lumen ( 134) verbunden ist und eine Instrumenten-Kanal-Öffnung (152), die mit einem Ende des genannten Instrumenten-Kanal-Rohr (150) in Verbindung steht.

8. Ein seitlich montierbares Überrohr (100) nach einem der Ansprüche 1 - 7, umfassend auch einen Sicherungsmechanismus (170) zur auswählbarer Sicherung des seitlich montierbaren Überrohrs (100) auf dem erwähnten Endoskop (130).

9. Ein seitlich montierbares Überrohr (100) nach Anspruch 8 und wobei der erwähnte Sicherungsmechanismus (170) ein doppelseitig anklebendes Band aufweist, welches zum auswählbaren Verbinden gegenüberliegender axialen Kanten (108, 110) des erwähnten Schlitzes (106) geeignet ist.

10. Ein seitlich montierbares Überrohr (100) nach einem der Ansprüche 1 - 9, umfassend auch eine Inflations-/Deflations-Funktionalität (147), die für auswählbares Aufblasen und Ausblasen des Ballons (120) wirksam ist.

## Revendications

1. Une sonde («overtube ») (100) installable à côté pour l'utilisation avec l'un endoscope (130), ladite sonde (100) comportant :
- Un manchon (102) formé par une fente (106) prolongeant complètement sur toute sa longueur, et une lumière de gonlement/dégonflement de ballon (132) en communication avec une ouverture de ballon (142) à l'emplacement intermédiaire de l'ouverture de ballon sur toute sa longueur ; et
- sélectivement un ballon gonflable/dégonflable (120) monté sur ledit manchon (120) dans une façon qu'il ne prolonge pas complétement à travers ladite fente (106) et aussi il est installable à côté ensemble avec ledit manchon (102) sur un endoscope (130), l'intérieur dudit ballon communiquant avec ladite ouverture de ballon (142) audit emplacement d'ouverture de ballon pour gonflement/dégonflement dudit ballon (120) sélectivement.

2. Une sonde («overtube ») (100) installable à côté selon la revendication 1, **caractérisé en ce que** ledit ballon gonflable/dégonflable (120), une fois gonflé, définit des surfaces (192, 194) de ballon gonflé touchant avec les uns les autres réciproquement, prolongeant généralement sur tous les bords (108, 110) de ladite fente (106).

3. Une sonde («overtube ») (100) installable à côté selon l'une ou l'autre des revendications 1 et 2, **caractérisé en ce que** ladite sonde (100) est configurée pour l'attachement démontable audit endoscope (130) quand ledit ballon (120) est gonflé, et donc empêchant le mouvement de glissement entre ladite sonde (100) et ledit endoscope (130).

4. Une sonde («overtube ») (100) installable à côté selon l'une ou l'autre des revendications 1 et 2, **caractérisé en ce que** ladite sonde (100) est configurée pour permettre un mouvement relative de glissement entre ladite sonde (100) et ledit endoscope (130) quand ledit ballon (120) est gonflé.

5. Une sonde («overtube ») (100) installable à côté selon l'une quelconque des revendications 1 à 4, **caractérisé en ce que** ladite sonde (100) de ballon gonflable/dégonflable (120) comporte au moins première et deuxième surfaces axiales d'étanchéité prolongeant généralement parallèles à ladite fente (106).

6. Une sonde («overtube ») (100) installable à côté selon l'une quelconque des revendications 1 à 5, **caractérisé en ce qu'**elle comporte aussi une lumière latérale (134) s'étendant au moins partiellement sur toute la longueur dudit manchon (102) et traversant ledit ballon (120).

7. Une sonde («overtube ») (100) installable à côté selon la revendication 6, **caractérisé en ce qu'**elle comporte aussi un tube de conduit (150) communiquant avec une pièce arrière de ladite lumière de côté (134) et un port de conduit d'instrument (152) communiquant avec une pièce d'extrémité dudit tube de conduit d'instrument (150).

8. Une sonde («overtube ») (100) installable à côté selon l'une quelconque des revendications 1 à 7, **caractérisé en ce qu'**elle comporte aussi un mécanisme de verrouillage (170) pour fixer ladite sonde (100) installable à côté sur ledit endoscope (130).

9. Une sonde («overtube ») (100) installable à côté selon la revendication 8, **caractérisé en ce que** ledit mécanisme de verrouillage (170) comporte une bande adhésive double-face adaptée sélectivement à adhérer les bords axiaux opposés (108, 110) de ladite fente (106).

10. Une sonde («overtube ») (100) installable à côté selon l'une quelconque des revendications 1 à 9, **caractérisé en ce qu'**elle comporte d'une fonctionnalité de gonflement/dégonflement (147) opérative pour sélective gonflement et dégonflement dudit ballon (120).
